(19) Europäisches Patentamt
European Patent Office
Office européen des brevets

(11) Veröffentlichungsnummer: **0 423 632 B1**

# (12) EUROPÄISCHE PATENTSCHRIFT

(45) Veröffentlichungstag der Patentschrift: **16.08.95**

(21) Anmeldenummer: **90119576.8**

(22) Anmeldetag: **12.10.90**

(51) Int. Cl.$^6$: **C07D 233/64**, C12Q 1/34, C12Q 1/42, C07D 263/32, C07D 277/24, C07D 277/28, C07H 15/26, C07F 9/6509, C07F 9/653, C07F 9/6539

## (54) Hydrolase-Substrate.

(30) Priorität: **17.10.89 US 422496**

(43) Veröffentlichungstag der Anmeldung: **24.04.91 Patentblatt 91/17**

(45) Bekanntmachung des Hinweises auf die Patenterteilung: **16.08.95 Patentblatt 95/33**

(84) Benannte Vertragsstaaten: **AT BE CH DE DK ES FR GB GR IT LI LU NL SE**

(56) Entgegenhaltungen:
**EP-A- 0 161 436** **EP-A- 0 167 973**
**EP-A- 0 243 066** **EP-A- 0 291 321**
**EP-A- 0 333 114** **EP-A- 0 347 139**

**CHEMICAL ABSTRACTS, Band 106, Nr. 11, 16. März 1987, Seite 612, Spalte 2, Zusammenfassung Nr. 84606v, Columbus, Ohio, US; & JP-A-61 229 868 (WAKO PURE CHEM. INDUSTR.) 14-10-1986**

(73) Patentinhaber: **BOEHRINGER MANNHEIM GMBH**

**D-68298 Mannheim (DE)**

(72) Erfinder: **Guder, Hans-Joachim, Dr. rer nat.**
**Schwattachweg 1**
**W-8120 Weilheim (DE)**
Erfinder: **Güthlein, Werner, Dr. rer nat.**
**Im Sennteich 31**
**W-6800 Mannheim 24 (DE)**
Erfinder: **Weckerle, Wolfgang, Dr. rer nat.**
**Auf dem Leimen 12**
**W-6718 Grünstadt (DE)**
Erfinder: **Berger, Johann, Dr.**
**Jägergasse 10**
**W-8127 Iffeldorf (DE)**
Erfinder: **Buck, Harvey, Dr.**
**9115 Hague Road**
**Indianapolis,**
**Indiana 46250 (US)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 423 632 B1

**CHEMICAL ABSTRACTS, Band 102, Nr. 25, 24. Juni 1985, Seite 322, Spalte 2, Zusammenfassung Nr. 217842k, Columbus, Ohio, US; & JP-A-59 193 353 (FUJI PHOTO FILM CO., LTD) 01-11-1984**

**CHEMICAL ABSTRACTS, Band 85, Nr. 21, 22. November 1976, Seite 538, Spalte 1, Zusammenfassung Nr. 159962s, Columbus, Ohio, US; D.T. CHAUDHARI et al.: "Synthesis and study of 4-(p-aminophenyl)-thiazoles for in vitro antitubercular activity and as useful chemotherapeutic intermediates", & BULL. HAFFKINE INST. 1976, 4(1), 8-15**

Erfinder: **Herrmann, Rupert, Dr. rer. nat.**
**In der Au 23**
**W-8120 Weilheim (DE)**

## Beschreibung

Gegenstand der Erfindung sind neue Hydrolase-Substrate, Verfahren zu ihrer Herstellung und Verfahren zum Nachweis von Substanzen mit Hydrolaseaktivität in einer Probe.

Hydrolasen sind Enzyme, die in jüngerer Zeit große Bedeutung erlangt haben. Einerseits spielen sie eine wichtige Rolle im Stoffwechsel von Pflanzen, Tieren und auch des Menschen. Weicht die Konzentration einer Hydrolase in einem dieser biologischen Systeme von der normalerweise darin vorhandenen Konzentration ab, so kann dies die Ursache einer Krankheit sein. Daher ist es eine Aufgabe der klinischen Diagnostik, bei Vorliegen einer Krankheit durch Bestimmung der Konzentration einer Hydrolase in Körperflüssigkeiten eventuelle Abweichungen vom Normalwert festzustellen. Dies geschieht zweckmäßig über die Bestimmung der Hydrolaseaktivität mittels einer Indikatorreaktion. Die zu untersuchende Probe wird dazu mit einem Substrat für die betreffende Hydrolase versetzt. Die aus dem Substrat gebildete Menge an Produkt ist ein Maß für die vorhandene Hydrolasekonzentration.

Andererseits werden Hydrolasen immer häufiger als Enzyme zur Markierung von immunologisch aktiven Verbindungen eingesetzt. Besonders breite Anwendung hat hierbei β-Galaktosidase zum Markieren von beispielsweise Antikörpern bei immunologischen Tests gefunden (Annals of Clinical Biochemistry 16, 221 (1979)). Tests dieser Art dienen dazu, den Gehalt eines immunologisch aktiven Analyten in einer Probe zu bestimmen. Sie sind so aufgebaut, daß die Konzentration des Analyten über einen passenden Immunpartner ermittelt wird, der als Markierung kovalent gebundene β-Galaktosidase trägt. Durch die Testführung wird erreicht, daß die Konzentration des Immunpartners direkt abhängig ist von der Konzentration des zu bestimmenden Analyten. Die Konzentration des markierten Immunpartners wird ebenfalls über eine Indikatorreaktion sichtbar gemacht. In der Indikatorreaktion wird der mit β-Galaktosidase markierte Immunpartner mit einem Substrat für β-Galaktosidase umgesetzt.

Die gebildete Menge an Produkt ist proportional zu der Konzentration des Immunpartners. Durch Vergleich mit den Werten einer Eichkurve, die mit Hilfe von Proben mit bekannter Analytkonzentration aufgenommen wird, kann eine unbekannte Analytkonzentration in einer Probe bestimmt werden.

Hydrolasen werden auch als Enzyme zur Markierung von Nukleinsäuren in Verfahren zum Nachweis von Nukleinsäuren eingesetzt. Ein solches Verfahren, welches β-Galaktosidase als Enzymmarkierung verwendet, ist in der DE-A-29 15 082 beschrieben. Auch hier wird die Menge an Markierung in einer Indikatorreaktion bestimmt.

Hydrolasen werden auch als Forschungsreagenzien verwendet. Auch hier ist es wichtig, die Konzentration der Enzyme genau und schnell bestimmen zu können. Im allgemeinen können hierzu ähnliche Methoden Einsatz finden, wie sie in der klinischen Diagnostik angewandt werden.

Das Substrat ist dabei meistens eine in der Probenflüssigkeit lösliche chemische Verbindung. Bei der Umsetzung mit der Hydrolase wird ein Produkt gebildet, welches sich in einer seiner Eigenschaften, beispielsweise einer charakteristischen Lichtabsorption, einer Lichtemission (Fluoreszenz usw., vom Substrat unterscheidet und dadurch nachgewiesen werden kann.

Bei den Testverfahren auf immunologisch aktive Substanzen unterscheidet man zwei technische Ausführungsformen. Bei einer häufig verwendeten Ausführungsform findet die Nachweisreaktion in einer Küvette statt, wie sie für photometrische Untersuchungen allgemein verwendet wird. Die Auswertung der Indikatorreaktion wird dann durch Messung der Absorption, Emission oder Radioaktivität in einem geeigneten Gerät vorgenommen.

Bei einer anderen Ausführungsform laufen die Reaktionen in einem oder mehreren Vliesen oder Filmen, welche Teil eines Testträgers sind, ab. Auf diesen Vliesen oder Filmen sind die benötigten Reagenzien aufgebracht. Die Menge an Produkt, das durch die Hydrolaseaktivität aus dem Hydrolasesubstrat während der Indikatorreaktion freigesetzt wird, kann dann in einem solchen Vlies oder Film direkt ermittelt werden. Ein Vorteil dieser Ausführungsform ist, daß meist mit nur einer einzigen Lösung, der flüssigen Probe, gearbeitet werden kann, was die Verfahrensführung erheblich erleichtert. Besonders einfach läßt sich die Menge an gebildetem Produkt durch Messung der Lichtabsorption bei einer bestimmten Wellenlänge verfolgen.

Hierfür geeignete Substrate sind die in der EP-A-01 46 866 beschriebenen Phenolsulfonphthaleinyl-β-D-galaktoside und ihre Derivate, aus denen durch Reaktion mit β-D-Galaktosidase Phenolsulfonphthaleinderivate freigesetzt werden. Während das Substrat eine gelbe Färbung aufweist, ist das Produkt rot gefärbt. Es findet also während der Indikatorreaktion ein allmählicher Farbumschlag von gelb über orange nach rot statt. Der Farbumschlag ist mit dem bloßen Auge nur sehr ungenau zu beurteilen, daher muß man zur Messung ein geeignetes Photometer benutzen.

Auch bei den in der EP-A-0 156 347 beschriebenen Resorufin-β-D-glykosiden handelt es sich um Substrate, bei deren Umsetzung mit einer Glykosidase ein Farbumschlag von gelb nach rot stattfindet. Es

hat sich herausgestellt, daß im Übergangsbereich zwischen gelb und rot besonders bei niedrigen β-Glykosidaseaktivitätendie visuelle Auswertung zu subjektiven Ergebnissen führt, weshalb auch hier zur Auswertung Geräte eingesetzt werden. Diese Geräte sind jedoch relativ kompliziert und daher teuer. Die Resorufinglykoside sind als Substrate für den Einsatz in Testträgern nicht gut geeignet, da das aus ihnen gebildete Resorufin ausblutet.

Hydrolasesubstrate, beispielsweise Methylumbelliferongalaktoside, bei deren Hydrolyse nur eine Änderung von Fluoreszenzeigenschaften stattfindet, sind ebenfalls für eine visuelle Auswertung ungeeignet. Auch das daraus gebildete Methylumbelliferon blutet auf Teststreifen leicht aus.

HHydrolasesubstrate eingesetzt werden, die bei der Reaktion mit einer Hydrolase in farbige Produkte übergehen. Bei diesen Nachweisreaktionen muß demnach nicht ein Farbumschlag, sondern das Entstehen einer Farbe beurteilt werden. Dies kann nämlich in einfacher Weise durch Vergleich der Farbintensität mit einem Farbton einer Farbskala geschehen. Auch farbenblinde Personen können einen solchen Test auswerten.

Ein solches Substrat ist 5-Brom-4-chlor-3-indolyl-β-galaktosid. Dieses Substrat ergibt nach Spaltung und Oxidation einen Farbstoff, der auf Testträgern nicht ausblutet. Das Substrat selbst ist aber wenig wasserlöslich. Ein darauf aufbauender Test ist für eine schnelle visuelle Auswertung somit weniger geeignet. Der Mechanismus, der zur Farbbildung führt, ist die oxidative Dimerisierung zweier 5-Brom-4-chlor-indolyleinheiten zu einem Indigofarbstoffderivat. Es sind demnach für die Bildung eines Farbstoffmoleküls zwei enzymatische Spaltungen von Substraten notwendig. Man erreicht damit also nur die Hälfte der theoretisch möglichen Sensitivität.

In Biochem. Z. 333, 209 (1960) wurden Verbindungen als Substrate vorgeschlagen, bei deren Hydrolyse Phenole freigesetzt werden. Diese bluten jedoch auf Testträgern leicht aus. Im Fall von Nitrophenol entwickelt sich während der Indikatorreaktion eine mehr oder weniger intensive Gelbfärbung. Die visuelle Auswertung im Bereich kleiner Konzentrationen ist bei der Wellenlänge der Nitrophenole sehr ungünstig.

Zur besseren Sichtbarmachung werden in einer zusätzlichen Reaktion die freigesetzten Phenole oxidativ mit Aminoantipyrin oder Methylbenzothiazolinonhydrazon gekoppelt (Anal. Biochem. 40, 281 (1971)) oder mit Diazoniumsalzen zu Azofarbstoffen (beispielsweise Histochemie 37, 89 (1973)) umgesetzt. Manche dieser Farbstoffe sind zwar für den Einsatz in Testträgern geeignet, da sie nicht so leicht ausbluten; der Einsatz dieser Kopplungsreaktion ist jedoch aus anderen Gründen von Nachteil. Zur Testführung auf einem Vlies oder einem Film müssen nämlich alle für diese zusätzliche Reaktion erforderlichen Reagenzien ebenfalls auf ein Vlies aufgebracht oder in einen Film eingebracht werden. Die große Anzahl von Reagenzien, wie beispielsweise Kopplungspartnern, kompliziert die Testführung und bringt zahlreiche Nachteile und Schwierigkeiten mit sich; so muß im Einzelfall darauf geachtet werden, daß die Reagenzien nicht schon vorzeitig miteinander oder unspezifisch mit anderen Komponenten der Probenlösung reagieren usw. So können Aminoantipyrin oder Methylbenzothiazolinonhydrazon auch mit Bestandteilen beispielsweise in untersuchtem Urin reagieren und so die Messung verfälschen. Andere Kopplungspartner, wie Diazoniumsalze, beeinträchtigen die Lagerstabilität erheblich. Auch ist bei der Wahl der Reagenzien zu berücksichtigen, daß sie nicht schon selbst eine Eigenfärbung aufweisen etc.

Ausblutungserscheinungen, die bei einigen der gebildeten Farbstoffe auf Testträgern auftreten, haben zum Beispiel zur Folge, daß die Farbintensität nicht an allen Stellen des Vlieses oder Films gleich ist, was zu Nachteilen bei der Auswertung führt. So werden beispielsweise die Meßwerte verfälscht.

Es bestand daher ein Bedarf an Hydrolasesubstraten, bei denen die geschilderten Nachteile vermieden werden und mit deren Hilfe Substanzen mit Hydrolase-Aktivität auch auf Testträgern in einfacher, schneller und zuverlässiger Weise nachgewiesen werden können.

Aufgabe der vorliegenden Erfindung war es, diesen Bedarf zu befriedigen.

Gelöst wird diese Aufgabe durch Verbindungen der Formel I

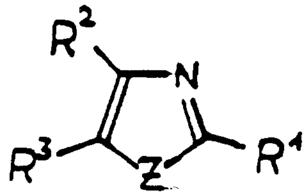

(I)

in der

Z Sauerstoff, Schwefel oder die Gruppe $>N-R^4$ bedeutet

$R^1$, $R^2$ und $R^3$ gleich oder verschieden sein können und Wasserstoff, eine Alkyl- oder Aralkylgruppe

oder einen aromatischen Rest bedeuten

$R^4$ Wasserstoff oder eine Alkylgruppe darstellt

und in der ein oder mehrere der Reste $R^1$, $R^2$ und $R^3$ einen durch die Gruppe

-O-X

substituierten aromatischen Rest bedeuten.

Die Alkylgruppen der Reste $R^1$, $R^2$, $R^3$ und $R^4$ weisen bevorzugt 1 - 6 Kohlenstoffatome auf. Besonders bevorzugt sind Alkylgruppen mit 1 - 4 Kohlenstoffatomen, ganz besonders bevorzugt ist der Methylrest.

Als Aralkylgruppe der Reste $R^1$, $R^2$ und $R^3$ sind Aralkylgruppen mit 7 bis 11 Kohlenstoffatomen, und besonders der Benzylrest, bevorzugt.

Ein aromatischer Rest in der Bedeutung der Reste $R^1$, $R^2$ und $R^3$ kann substituiert oder unsubstituiert sein und weist bevorzugt 6 bis 12 Kohlenstoffatome auf.

Als unsubstituierter Rest ist der Phenyl- und der Naphthylrest bevorzugt. Weitere mögliche Reste sind beispielsweise Heteroarylreste, wie beispielsweise der Furyl- und der Pyridylrest.

Substituierte aromatische Reste sind bevorzugt der Phenyl- und der Naphthylrest, bei denen ein oder mehrere Wasserstoffatome durch Halogen, einen Hydroxyl-, Alkoxy-, Alkyl-, Amino-, Mono- oder Dialkylamino-, Carboxyl-, Carbamoyl-, Alkoxycarbonyl-, Alkylcarbonyl-, Formyl- oder Sulforest oder die Gruppe -O-X ersetzt sind. Ein aromatischer Rest kann auch verschiedene dieser Substituenten gleichzeitig tragen. Als Halogen sind bevorzugt Chlor, Brom und Jod, besonders bevorzugt Chlor.

Alkylgruppen des aromatischen Rests und Alkylreste in den einzelnen Substituenten des aromatischen Rests weisen bevorzugt 1 - 6, insbesondere 1 - 4 Kohlenstoffatome auf und können ihrerseits substituiert oder unsubstituiert sein. Besonders bevorzugt ist der Methylrest. Als Substituenten der Alkylgruppen sind bevorzugt Halogen oder eine Hydroxy-, Alkoxy-, Carboxy-, Sulfo-, Phosphono- oder Morpholinogruppe.

Die Carboxyl-, Sulfo- und Phosphonogruppen können auch verestert sein, bevorzugt mit Methanol oder Ethanol.

Bevorzugte Substitutionsstellen der aromatischen Reste sind die meta- und die para-Position.

Besonders bevorzugte Verbindungen der Formel I sind solche, in denen einer der Reste $R^1$, $R^2$ und $R^3$ ein durch die Gruppe -O-X substituierter Phenylrest und ein anderer oder beide anderen Reste aromatische Reste sind, die einen oder mehrere elektronenschiebende Substituenten, wie beispielsweise Hydroxygruppen, substituierte oder nicht substituierte Aminogruppen oder Alkoxygruppen usw. aufweisen. Bevorzugte Substitutionsstellen für die elektronenschiebenden Substituenten sind die para-Positionen. Bevorzugte Substituenten der Aminogruppen sind Alkylgruppen mit 1 - 4 Kohlenstoffatomen, besonders bevorzugt ist die Methyl- und Ethylgruppe, oder Acylgruppen mit 1 - 6 Kohlenstoffatomen, bevorzugt der Acetylrest. Bevorzugt sind zweifach substituierte Aminogruppen.

Der aromatische Rest, der die Gruppe -O-X trägt, kann ebenfalls zusätzlich substituiert sein. Bevorzugte Substituenten sind der Alkoxy- und der Alkylrest. Insbesondere ist der Methoxyrest bevorzugt. Als aromatischer Rest in der Bedeutung der Reste $R^1$, $R^2$ und $R^3$, welcher keine Gruppe -O-X trägt, ist bevorzugt der 4-Dimethylaminophenyl-, 9-Julolidino-, 4-C1-C4-Alkoxyphenylrest, besonders bevorzugt der 4-Methoxyphenylrest, sowie 6-N-Methyl-1,2,3,4-tetrahydrochinolino-Rest, wobei die Methylsubstituenten Carboxyl-, Phosphono- oder Sulfo-Gruppen tragen können.

In der Gruppe -O-X bedeutet

O Sauerstoff und

X einen Glykosyl-, Phosphat- oder Acylrest.

Glykosylreste der Gruppe X sind Mono-, Oligo- und Polysaccharidreste der Hexosepyranosidreihe. Bevorzugt sind Monosaccharidreste, beispielsweise der Glukosyl- und der Galaktosylrest aber auch der Glukosaminrest, der gewünschtenfalls acyliert, bevorzugt N-acetyliert sein kann. Die Bindung des Glykosidrestes kann $\alpha$- oder $\beta$-glykosidisch sein.

Acylreste der Gruppe X weisen bevorzugt 1 bis 20 Kohlenstoffatome auf und können gesättigt oder ungesättigt, geradkettig oder verzweigt sein. Besonders bevorzugt ist der Acetylrest.

Zu den Acylresten zählen auch die Aminoacylreste. Bevorzugt als Aminoacylreste sind die über die Carboxylfunktion gebundenen Reste der natürlich vorkommenden $\alpha$-L-Aminosäuren. Dabei können die freien polaren Reste der Aminosäuren, wie beispielsweise die Aminofunktionen, durch geeignete Schutzgruppen geschützte sein. Ein solcher Aminosäurerest ist beispielsweise der Alanylrest, der an der Aminofunktion einen Tosylrest trägt.

Der Phosphatrest ist der Rest $-PO_3H_2$ und seine Salze. Als Kationen in diesen Salzen können alle positiv geladenen Ionen dienen. Bevorzugt sind Alkali-, Erdalkali- und Ammoniumionen; besonders bevorzugt sind Natrium-, Kalium-, Magnesium- und Calciumionen.

In den Verbindungen der Formel I können alle Reste $R^1$, $R^2$ und $R^3$ einen durch die Gruppe -O-X substituierten aromatischen Rest bedeuten. Bevorzugt sind jedoch Verbindungen der Formel I, in denen höchstens zwei, ganz besonders bevorzugt nur einer, der Reste $R^1$, $R^2$ und $R^3$ einen solchen aromatischen Rest darstellen.

Wenn Z Sauerstoff oder Schwefel bedeutet, dann sind davon Verbindungen bevorzugt, bei denen $R^1$ und $R^2$ diese Reste sind, jedoch $R^3$ nicht Wasserstoff bedeutet.

Wenn Z Sauerstoff oder Schwefel bedeutet, sind Verbindungen der Formel I besonders bevorzugt, in denen nur einer der Reste $R^1$ und $R^2$ ein durch die Gruppe -O-X substituierter aromatischer Rest bedeutet. Als die zwei Reste aus der Gruppe $R^1$, $R^2$ und $R^3$, die dann keinen durch die Gruppe -O-X substituierten aromatischen Rest darstellen, sind solche bevorzugt, die nicht solche aromatische Reste sind, welche beide Hydroxygruppen oder unsubstituierte Aminogruppen tragen. Jedoch sind beispielsweise als diese beiden Reste solche nicht bevorzugt, von denen einer einen durch eine Hydroxygruppe, der andere einen durch eine substituierte Aminogruppe substituierten aromatischen Rest bedeutet.

Wenn Z die Gruppe $>$N-$R^4$ bedeutet, sind Verbindungen der Formel I besonders bevorzugt, in denen nur einer der Reste $R^1$, $R^2$ und $R^3$ ein durch die Gruppe -O-X substituierter aromatischer Rest bedeutet.

- Ist $R^1$ dieser Rest, so sind die Verbindungen bevorzugt, in denen nicht gleichzeitig $R^2$ und $R^3$ einen durch eine Hydroxy- oder eine Aminogruppe substituierten aromatischen Rest bedeuten, oder einer der beiden Reste einen durch eine Aminogruppe, der andere einen durch eine Hydroxygruppe substituierten aromatischen Rest bedeutet.
- Ist $R^2$ oder $R^3$ dieser Rest, so sind Verbindungen bevorzugt, in denen nicht gleichzeitig $R^1$ und der andere Rest $R^3$ oder $R^2$ einen durch eine Hydroxy- oder eine Aminogruppe substituierten aromatischen Rest bedeuten.

Die Verbindungen der Formel I sind neue Verbindungen.

Sie können hergestellt werden durch Reaktion von Verbindungen der Formel II,

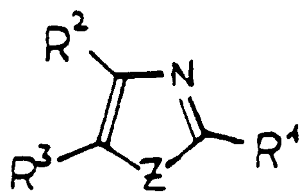

(II)

in der

Z, $R^1$, $R^2$, $R^3$ und $R^4$ die oben angegebene Bedeutung haben, wobei jedoch ein oder mehrere der Reste $R^1$, $R^2$ und $R^3$ einen durch die Gruppe -O-H anstelle der Gruppe -O-X substituierten Rest bedeuten,

mit Verbindungen der Formel III

X-Y (III)

in denen X die oben angegebene Bedeutung hat und Y eine reaktive Gruppe bedeutet.

Für den Fall, daß X ein Glykosylrest ist, können dazu Verfahren angewandt werden, wie sie für ähnliche Verbindungen aus der Kohlenhydratchemie bekannt sind.

Dazu werden Verbindungen der Formel II mit einem aktivierten Zucker der Formel III umgesetzt. Als besonders geeignet haben sich dabei Verbindungen erwiesen, bei denen Y eine gut austretende nucleophile Gruppe, beispielsweise Halogen, insbesondere Chlor oder Brom, oder ein Sulfonyloxyrest, insbesondere ein Toluolsulfonyloxy- oder Methansulfonyloxyrest, bedeuten. Es hat sich dabei als zweckmäßig erwiesen, freie Hydroxy- oder Aminogruppen während der Reaktion durch eine geeignete Schutzgruppe geschützt zu halten. Die Gruppe -O-H, die dabei in die Gruppe -O-X überführt werden soll, darf dabei allerdings keine Schutzgruppe tragen.

Für den Fall, daß X ein Acylrest ist, können Verfahren angewandt werden, wie sie zur Herstellung von Estern aus Alkoholen sowie speziell Phenolen durch Reaktion mit organischen Carbonsäuren allgemein bekannt sind. Funktionelle Gruppen der Aminosäuren werden vor der Veresterung zweckmäßigerweise durch eine geeignete Schutzgruppe maskiert. Nach der Reaktion können die Schutzgruppen wieder abgespalten werden. Die reaktive Gruppe Y ist bevorzugt eine gut austretende nucleophile Gruppe. Vorteilhaft sind hierbei besonders Halogen, Alkoholat und Carboxylat.

Für die Herstellung von Verbindungen der Formel I, in denen X der Phosphatrest ist, sind besonders Verbindungen der Formel III geeignet, in denen X der Rest $-POW_2$ oder $-PW_4$, in denen W eine gute Abgangsgruppe, insbesondere Alkoholat mit 1 - 4 C-Atomen oder Chlorid, Bromid oder Jodid bedeutet, und Y der Rest Halogenid, bevorzugt Chlorid, Bromid oder Jodid, oder Alkoholat bedeutet, besonders bevorzugt als Verbindung der Formel X-Y ist Phosphoroxitrichlorid. Auch hier ist es zweckmäßig, freie Hydroxy- und Aminogruppen mit Ausnahme der in die Gruppe -O-X zu überführenden Hydroxygruppe durch nach der Reaktion abspaltbare Schutzgruppen zu maskieren. Verbindungen, in denen X der Rest $-POW_2$ oder $-PW_4$ bedeutet, können durch Hydrolyse in bekannter Weise in die Phosphate überführt werden.

Die Salze der Phosphate können in bekannter Weise, beispielsweise durch Ionenaustausch, aus den freien Säuren hergestellt werden.

Die Verbindungen der Formel II und III sind bekannte Verbindungen oder können analog zu bekannten Verfahren der organischen Synthese hergestellt werden. Insbesondere sind Triarylimidazole aus der DE-A-27 35 690 bekannt. Die EP-B-0 161 436 und die EP-A-0 167 973 beschreiben ebenfalls Verbindungen der Formel II. Die Verwendung erstreckt sich auf den Nachweis von Hydroperoxid.

Die erfindungsgemäßen Substanzen der Formel I können beispielsweise auch hergestellt werden, indem man in an sich bekannter Weise entweder

a) für den Fall, daß Z die Gruppe $>N-R^4$ und X ein Glykosylrest bedeutet, ein α-Diketon der Formel IV,

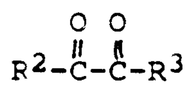

(IV)

in der $R^2$ und $R^3$ die oben angegebene Bedeutung besitzen, mit einem Aldehyd der Formel V,

$O=CH-R^1$ (V)

in der
$R^1$ die oben angegebene Bedeutung besitzt und mit Ammoniak oder einen Amin der Formel VI

$NH_2R^4$ (VI)

in der
$R^4$ die oben angegebene Bedeutung hat, in Eisessig kondensiert oder

b) für den Fall, daß Z die Gruppe $>N-H$ und X ein Glykosylrest bedeutet, ein α-Ketoxim der Formel VII,

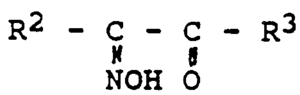

(VII)

in der
$R_2$ und $R_3$ die oben angegebene Bedeutung besitzen, mit einem Aldehyd der Formel V und Ammoniak oder einem Amin der Formel VI zu einer Verbindung der Formel VII a)

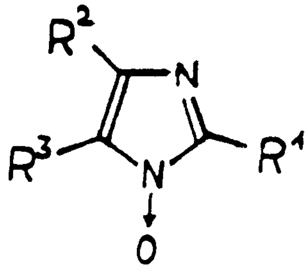

(VII a)

in der

$R^1$, $R^2$ und $R^3$ die in Formel II angegebene Bedeutung haben, umsetzt, und diese Verbindung reduziert oder

c) für den Fall, daß Z die Gruppe >N-H und X ein Glykosylrest bedeutet ein α-Halogenketon der Formel VIII,

$R^2$-CO-CHR$^3$Hal (VIII)

in der

$R^2$ und $R^3$ die oben angegebene Bedeutung besitzen und Hal Fluor, Chlor oder Brom darstellt, mit einem Amidin der Formel IX

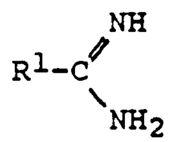

(IX)

in der

$R^1$ die oben angegebene Bedeutung besitzt, in alkalischem Medium umsetzt oder

d) für den Fall, daß Z Sauerstoff oder die Gruppe >N-H bedeutet, eine Verbindung der Formel X,

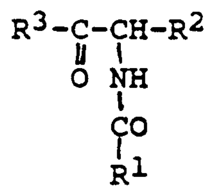

(X)

in der

$R^1$, $R^2$ und $R^3$ die oben angegebene Bedeutung haben, mit einer Lewis-Säure oder Pentasulfid umsetzt

oder

e) für den Fall, daß Z Sauerstoff bedeutet, eine Verbindung der Formel XI,

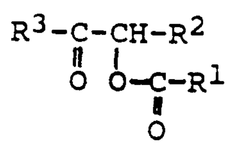

(XI)

in der

$R^1$, $R^2$ und $R^3$ die angegebene Bedeutung haben,

mit Ammoniumacetat in Eisessig umsetzt, oder

f) für den Fall, daß Z Schwefel und X ein Glykosylrest bedeutet,

ein Thioamid der Formel XII,

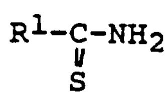

(XII)

in der

$R^1$ die oben angegebene Bedeutung hat,

mit einem α-Halogenketon der Formel VIII, in der $R^2$ und $R^3$ die oben angegebene Bedeutung haben und Hal Fluor, Chlor oder Brom darstellt, umsetzt

und anschließend, falls erforderlich, die erhaltenen Verbindungen in Verbindungen der Formel I überführt

sowie entsprechende Basen in Salze oder Salze in Basen umwandelt.

In diesen Verfahren können gegebenenfalls vorhandene reaktive funktionelle Gruppen, an denen keine Reaktion stattfinden soll, durch bekannte Schutzgruppen geschützt werden, die später wieder abgespalten werden können.

Die Überführung der nach dem Verfahren a) bis f) erhaltenen Verbindungen in Verbindungen der Formel I geschieht z. B. durch katalytische Hydrierung (Nitro --> Amin), Abspaltung von Schutzgruppen durch Hydrierung mit Pd/C (O-Benzyl --> Hydroxyl), Hydrierung von Heterocyclen (Furanyl --> Tetrahydrofuranyl), Spaltung von Heterocyclen durch Hydrierung (Tetrahydrofuranyl --> 4-Hydroxybutyl), reduktive Alkylierung von Aminen (Amine --> Dialkylamine).

Durch Umsetzung von 4-Amino-Verbindungen der Formel I mit Halogen-methan- bzw. 2-ethan-carbonsäure, -Sulfonsäuren sowie -Phosphonsäuren bzw. ihren Salzen in DMF erhält man entsprechende N-alkylamino-methan- bzw. 2-ethan-Säurereste.

Die Reduktion von N-Oxiden der Formel VII a) kann beispielsweise mit Zink/Essigsäure oder katalytisch erregtem Wasserstoff geschehen.

Die Synthese von Verbindungen der Formel I kann auch gemäß J. Org. Chem. 2, 319 (1937) bzw. Z. Chem. 10, 431 (1970) und 11, 10 (1971) erfolgen.

Überraschenderweise eignen sich die neuen Verbindungen der Formel I gemäß der vorliegenden Erfindung hervorragend als Hydrolasesubstrate. Sie sind wasserlöslich, farblos und stabil, solange sie nicht mit einer Hydrolase in Kontakt kommen.

Ist eine außergewöhnlich gute Wasserlöslichkeit gewünscht, so verwendet man zweckmäßigerweise Verbindungen der Formel I, in denen einer oder mehrere polare Reste, wie beispielsweise Carboxyl-, Sulfo- oder Phosphonogruppen enthalten sind.

Die Löslichkeit der Verbindungen der Formel I kann auch durch Erniedrigung der Polarität des Reaktionsmediums, beispielsweise durch Zusatz von organischen Lösungsmitteln, wie Alkoholen, Dimethylsulfoxid oder Detergentien, erreicht werden.

Als Hydrolasesubstrate im erfindungsgemäßen Verfahren sind insbesondere diejenigen Verbindungen der Formel I geeignet, bei denen der Molekülteil X einem Molekülteil des natürlichen Substrats der entsprechenden Hydrolase analog ist. So sind beispielsweise als β- Galaktosidasesubstrate die Verbindungen der Formel I, in denen X ein β-Galaktosylrest bedeutet, als Esterasesubstrate die, in denen X einen Acylrest bedeutet und als Phosphatasesubstrat die Verbindungen der Formel I, in denen X ein Phosphatrest ist, besonders geeignet.

Ebenfalls Gegenstand der Erfindung ist ein Verfahren zum Nachweis von Substanzen mit Hydrolase-Aktivität in einer Probe durch Mischen der Probe mit einem Hydrolasesubstrat sowie einem Oxidationsmittel und Auswertung der entstehenden Farbintensität, dadurch gekennzeichnet, daß als Hydrolasesubstrat mindestens eine Verbindung der Formel I verwendet wird.

Substanzen mit Hydrolaseaktivität sind Substanzen, die chemische Verbindungen unter Verbrauch von Wasser in zwei Produkte spalten können. Dazu gehören natürlich vorkommende und künstlich erzeugte Hydrolasen der Enzymhauptklasse 3. Als besonders geeignet hat sich das erfindungsgemäße Verfahren zum Nachweis von Glykosidasen, bevorzugt Glukosidasen und Galaktosidasen, und Esterasen, bevorzugt Lipasen und Phosphatasen,besonders bevorzugt alkalische Phosphatase oder Schweineleber-Esterase, erwiesen.

Unter Substanzen mit Hydrolaseaktivität werden jedoch auch Verbindungen dieser Hydrolasen mit anderen chemischen Verbindungen, beispielsweise immunologisch aktiven Verbindungen oder Nukleinsäuren verstanden. Zu den immunologisch aktiven Verbindungen gehören beispielsweise Haptene, Antigene und Antikörper und Immunkomplexe. Jedoch zählen dazu genausogut Fragmente von Antikörpern, wie das Fab- oder $F(ab')_2$-Fragment. Diese Verbindungen der Hydrolasen mit immunologisch aktiven Verbindungen werden oft auch als Hydrolase-Konjugate bezeichnet. Die Hydrolase dient dabei zur Markierung der immunologisch aktiven Verbindung. Besonders geeignet ist das erfindungsgemäße Verfahren zum Nachweis von immunologisch aktiven Verbindungen mit β-Galaktosidase, β-Glukosidase sowie alkalischer oder saurer Phosphatase als Markierung.

Als Hydrolasesubstrat wird eine Verbindung bezeichnet, wenn sie von der betreffenden Substanz mit Hydrolaseaktivität umgesetzt wird. Aus dem Substrat werden unter Aufnahme von Wasser zwei Produkte gebildet. Die Reaktion befolgt dabei die in der Enzymkinetik bekannten Regeln. Voraussetzung ist, daß die Substrate zumindest zu einem großen Anteil in der die Substanz mit Hydrolaseaktivität enthaltenden Lösung löslich sind.

Unter einer Probe wird bevorzugt eine feste oder flüssige Probe verstanden.

Im Falle einer festen Probe kann man zwischen löslichen und im wesentlichen nicht löslichen Proben unterscheiden.

Eine lösliche Probe wird zweckmäßig zum Nachweis in eine flüssige Probe überführt:

Unter einer im wesentlichen nicht löslichen Probe wird bevorzugt ein Feststoff verstanden, auf dessen äußerer oder innerer Oberfläche eine Substanz mit Hydrolaseaktivität gebunden ist. Die Art der Bindung ist für das erfindungsgemäße Verfahren nicht von Bedeutung. Diese Bindung kann beispielsweise sowohl kovalent als auch ionisch oder adsorptiv oder auch biospezifisch sein. Biospezifische Bindungen sind Bindungen zwischen biologischen Bindungspartnern, wie beispielsweise die Bindungen zwischen als Antigen oder Hapten wirkenden Substanzen und Antikörpern, Biotin und Avidin bzw. Streptavidin, komplementären Nucleinsäuren usw.

Unter einer flüssigen Probe, in der die Substanzen mit Hydrolase-Aktivität nachgewiesen werden sollen, werden im wesentlichen wässrige Lösungen verstanden. Diese Lösungen können beispielsweise Lösungen einer Hydrolase in Wasser sein. Oft werden diesen Lösungen Beimengungen, wie Salze, Detergentien etc., beispielsweise zur Erhöhung der Lagerstabilität, zugegeben. Auch in solchen Lösungen kann das erfindungsgemäße Verfahren angewandt werden. Die flüssige Probe kann auch eine Körperflüssigkeit oder eine daraus durch Zugabe oder Abtrennung von Bestandteilen erhaltene Flüssigkeit sein. Dazu gehören beispielsweise Blut, Blutplasma, Serum oder Urin.

Die flüssige Probe kann bevorzugt auch eine Flüssigkeit sein, wie sie im Verlauf von immunologischen Testverfahren entsteht. Dieses sind beispielsweise die in Annals of Clinical Biochemistry 16, 221 (1979) beschriebenen und deren vorteilhafte Fortentwicklungen, die dem Fachmann auf dem Gebiet von Immunoassays bekannt sind. Auch in solchen Flüssigkeiten können Substanzen mit Hydrolase-Aktivität mit dem erfindungsgemäßen Verfahren nachgewiesen werden. Diese Lösungen enthalten meistens Puffersubstanzen, Stabilisatoren, Netzmittel etc., die den Nachweis jedoch nicht entscheidend stören.

Die Konzentration an Substanzen mit Hydrolase-Aktivität kann vorteilhaft in einem Bereich von $10^{-6}$ bis $10^{-15}$ mol/l, bevorzugt von $10^{-6}$ bis zu $10^{-12}$ mol/l bestimmt werden.

Das Verfahren ist geeignet für Probelösungen, in denen der pH-Wert zwischen 5 und 11, vorzugsweise zwischen 6 und 10 liegt. Der optimale pH-Wert, bei dem das Verfahren durchgeführt wird, hängt von der verwendeten Hydrolase ab. Für die schnelle Durchführung des Verfahrens ist es zweckmäßig, bei oder in der Nähe des pH-Wertes zu arbeiten, bei dem die Hydrolase ein Aktivitätsmaximum aufweist. Außerhalb dieses Bereiches kann merkliche nicht enzymatische Hydrolyse bzw. Inaktivierung der Hydrolase stattfinden.

Die Nachweisreaktion wird bevorzugt in einem saug- oder quellfähigen Träger durchgeführt.

Solche Träger sind beispielsweise Vliese oder Filme. Bevorzugt sind Vliese. Unter Vliesen wird ein papierähnlich aus Fasern aufgebautes Material verstanden. Als Fasermaterial finden bevorzugt Cellulose, Kunststoffe oder Gemische davon Verwendung. Geeignet sind auch schwammartige oder/und poröse Träger.

Die Nachweisreaktion kann jedoch auch in einem Gefäß beliebiger Form, beispielsweise einer Küvette oder auch auf einer Mikrotiterplatte, durchgeführt werden. Wenn der Reaktionsverlauf mittels Absorptionsphotometer verfolgt werden soll, sind solche Verbindungen der Formel I besonders geeignet, bei denen Produkte gebildet werden, die im Reaktionsmedium löslich sind. Eine Möglichkeit ist die Verwendung von Verbindungen der Formel I, die je nach Polarität des Reaktionsmediums eine oder mehrere polare Gruppen, beispielsweise eine Carboxyl- oder Sulfogruppe aufweisen und deren Reste keine stark lipophilen Eigenschaften aufweisen. Eine weitere Möglichkeit zum Nachweis von Substanzen mit Hydrolaseaktivität in Lösung ist der Zusatz von die Polarität des Reaktionsmediums erniedrigenden Lösungsmitteln, wie beispielsweise organischen Lösungsmitteln. Dadurch, daß man den Nachteil dieser zusätzlichen Komponente in Kauf nimmt, wird die Löslichkeit der Hydrolyseprodukte erhöht.

Für die Entwicklung einer Farbe während der Indikatorreaktion ist das Vorhandensein eines Oxidationsmittels erforderlich. Als Oxidationsmittel können solche Substanzen oder Substanzgemische dienen, deren Oxidationspotential oberhalb des Wertes für die freigesetzte Verbindung der Formel II liegt. Als besonders vorteilhaft haben sich beispielsweise Kaliumhexacyanoferrat-III, Perborat/Peroxidase, Peroxid/Peroxidase, Tetrazoliumsalze oder Sauerstoff/Bilirubinoxidase erwiesen.

Zur Durchführung des erfindungsgemäßen Verfahrens wird die Probe mit mindestens einer, bevorzugt einer, Verbindung der Formel I als Hydrolasesubstrat und einem Oxidationsmittel vermischt.

Die Vermischung der Probe mit dem Hydrolasesubstrat kann auf unterschiedliche Art und Weise erfolgen.

Das Vermischen der Probe mit dem Hydrolasesubstrat und dem Oxidationsmittel kann gleichzeitig oder nacheinander geschehen.

Im Falle einer flüssigen Probe, beispielsweise einer Lösung der Substanz mit Hydrolaseaktivität können das Hydrolasesubstrat bzw. das Oxidationsmittel in Form eines Feststoffs, beispielsweise eines Pulvers, einer Tablette, eines Lyophilisats u. ä., oder in Form einer Lösung der Probe zugesetzt werden.

Findet die Nachweisreaktion in einem saug- oder quellfähigen Träger statt, so hat es sich als besonders zweckmäßig erwiesen, die Probe auf einen Träger aufzugeben, auf den das Hydrolasesubstrat sowie gegebenenfalls das Oxidationsmittel und Zusatzstoffe imprägniert sind. Zur Imprägnierung wird eine Lösung der genannten Stoffe hergestellt und damit das Trägermaterial getränkt. Anschließend wird das getränkte Trägermaterial getrocknet.

Für den Fall, daß der Träger ein Film ist, werden bevorzugt das Hydrolasesubstrat sowie gegebenenfalls Oxidationsmittel und Zusatzstoffe schon bei dessen Herstellung in den Film inkorporiert.

Falls das Oxidationsmittel nicht zusammen mit dem Hydrolasesubstrat vorliegt, wird das Oxidationsmittel vor oder nach der Vermischung der Probe mit dem Hydrolasesubstrat mit der Probe gemischt.

Findet die Nachweisreaktion in einem Gefäß statt, so hat es sich als besonders zweckmäßig erwiesen, das Hydrolasesubstrat in fester Form oder als Lösung, evtl. vermischt mit dem Oxidationsmittel und mit Zusatzstoffen, einzusetzen. Auch hier können das Oxidationsmittel bzw. Zusatzstoffe getrennt zugegeben werden.

Im Fall einer festen Probe, beispielsweise wenn die Substanz mit Hydrolaseaktivität an ein Trägermaterial gebunden ist, ist es zweckmäßig, das Hydrolasesubstrat oder/und das Oxidationsmittel der Probe in Form einer Lösung zuzusetzen; es ist jedoch beispielsweise auch möglich, die Komponenten zunächst zu vermischen und dann eine Flüssigkeit zur Herstellung einer Lösung zuzugeben.

Wichtig für den Erfolg des Nachweisverfahrens ist, daß das Hydrolasesubstrat mit der in der Probe vorhandenen Substanz mit Hydrolaseaktivität so in Kontakt treten kann, daß die Enzymreaktion ablaufen kann.

Während der Indikatorreaktion bildet sich eine kräftige Farbe, die je nach Substitutionsmuster von rot bis blau reicht. Die Farbintensität kann nach bekannten Verfahren mit den Photometern, insbesondere auch Reflexionsphotometern, gemessen werden. Dazu wird Licht der Wellenlänge eingestrahlt, welches das Produkt der Indikatorreaktion absorbieren kann. Bevorzugt ist eine Wellenlänge zwischen 500 und 700 nm, besonders bevorzugt zwischen 520 und 680 nm.

Die Farbintensität wird mit den Werten einer Eichkurve verglichen, die man durch Messung der Werte für Proben mit bekannten Konzentrationen an Substanzen mit Hydrolase-Aktivität erhält, und jeder Intensität eine bestimmte Konzentration zuordnet. Dieser Vergleich wird zweckmäßig mit Hilfe eines Rechners vorgenommen.

Dadurch, daß kein Farbumschlag, sondern eine Färbentwicklung beobachtet werden muß, ist die Auswertung aber auch durch das menschliche Auge möglich. Dies hat den Vorteil, daß kein Gerät verwendet werden muß. So entfallen auch mögliche Fehlerquellen, wie falsche Bedienung des Geräts etc.

Das erfindungsgemäße Verfahren kann zum qualitativen, aber auch zum quantitativen Nachweis von Substanzen mit Hydrolaseaktivität angewandt werden. Zur qualitativen Auswertung wird beobachtet, ob sich eine Verfärbung ergeben hat. Zur quantitativen Bestimmung wird zu einem festgelegten Zeitpunkt visuell die Farbe mit einer Farbskala verglichen, die jeder Farbe eine bestimmte Konzentration zuordnet.

Bei der Verwendung von erfindungsgemäßen Hydrolasesubstraten entstehen besonders intensiv gefärbte Verbindungen. In Abhängigkeit vom Substitutionsmuster lassen sich Verbindungen mit Farben von blau bis rot erzielen. Es resultiert eine große Anwendungsbreite.

Das erfindungsgemäße Verfahren kann insbesondere auf Testträgern mit besonderen Vorteilen angewandt werden. Vorteile sind beispielsweise, daß das Verfahren einfach und billig ist und dennoch zuverlässige Ergebnisse liefert. Einfach ist das Verfahren beispielsweise dadurch, daß es auch visuell ausgewertet werden kann; dadurch entfallen teure Geräte. Es ist besonders zuverlässig, da es nicht zu Ausblutungserscheinungen auf Testträgern führt. Dies ist von großem Vorteil.

Unter Testträgern versteht man Mittel zum Nachweis von Substanzen durch Ausführung eines Tests. Sie bestehen üblicherweise hauptsächlich aus einer Grundplatte oder Folie, auf welcher die für den Test erforderlichen Reagenzien meist mittels Vliesen oder Filmen angebracht sind.

Im erfindungsgemäßen Verfahren werden bevorzugt keine weiteren Kopplungskomponenten, wie beispielsweise Diazoniumsalze, eingesetzt; da diese zu Nebenreaktionen bzw. Stabilitätsverlusten führen. Bei der Farbbildung ist nur eine Verbindung der Formel I beteiligt. Dadurch werden die Nachteile, die bei Kondensationsvorgängen zwischen zwei Verbindungen vorkommen, und wie sie bei Verbindungen des Indoltyps vorliegen, vermieden.

Bei immunologischen Testverfahren fallen ebenfalls Proben speziell von Hydrolasekonjugaten an, die auf Ihren Gehalt an Substanzen mit Hydrolaseaktivität hin untersucht werden sollen.

Besondere Verwendung kann das erfindungsgemäße Verfahren zum Nachweis einer Substanz mit Hydrolaseaktivität daher beispielsweise in immunologischen Verfahren zum Nachweis eines Analyten finden. Solche Verfahren sind dem Fachmann auf dem Gebiet der Immunoassays bekannt (beispielsweise Annals of Clinical Chemistry 16, 221 (1979)). Diese Verfahren werden dahingehend abgewandelt, daß der

darin erforderliche Nachweis der hydrolasemarkierten immunologisch aktiven Verbindungen mittels des erfindungsgemäßen Verfahrens durchgeführt wird. Beispielhaft seien erwähnt:

Für den Fall, daß der Analyt ein Antigen oder Hapten ist, haben sich folgende Verfahren bewährt:

- Die den Analyten enthaltende Probenlösung wird mit einem Überschuß an Konjugat aus einem Antikörper gegen den Analyten und einer Hydrolase versetzt. Es bildet sich ein Immunkomplex aus Analyt und Konjugat. Der Überschuß an Konjugat wird durch Immunreaktion an einen Träger gebunden, an den ein Überschuß an Analyt oder einer analytanalogen Verbindung gebunden ist. Die das Konjugat aus Hydrolase und Immunkomplex enthaltende Lösung wird vom Träger getrennt und gemäß dem erfindungsgemäßen Verfahren behandelt. Man erhält ein Versuchsergebnis für das Hydrolasekonjugat, aus welchem man auf die Anwesenheit bzw. auf die Menge des Analyten schließen kann. Eine andere Möglichkeit ist der Nachweis des an den Träger gebundenen Konjugats aus Hydrolase und Antikörper. Auch dieser ist nach dem erfindungsgemäßen Verfahren möglich.
- Die den Analyten enthaltende Probenlösung wird mit einem Überschuß von Konjugat aus einem Antikörper gegen den Analyten und einer Hydrolase versetzt. Es bildet sich ein Immunkomplex aus Analyt und Konjugat. Der Überschuß an Konjugat wird durch Immunreaktion an einen Träger gebunden, an den ein Überschuß eines weiteren Antikörpers gegen den Analyten gebunden ist. Die den Überschuß des Konjugats aus Hydrolase und Antikörper enthaltende Lösung wird vom Träger getrennt und nach dem erfindungsgemäßen Verfahren entweder das an den Träger gebundene Konjugat aus Immunkomplex und Hydrolase oder das in der Lösung enthaltene Konjugat aus Antikörper und Hydrolase nachgewiesen.
- Die den Analyten enthaltende Probenlösung wird mit einer bekannten Menge an Konjugat aus Analyt oder Analytanalogon und einer Hydrolase versetzt. Das Gemisch wird auf einen Träger aufgegeben, an welchen ein bekannter Unterschuß eines Antikörpers gegen den Analyten und das Analytanalogon bezogen auf die Summe aus Analyt und Konjugat gebunden ist. Ein Teil des Analyten und des Konjugats wird an den Träger gebunden. Nach dem erfindungsgemäßen Verfahren kann nach Abtrennung der Lösung entweder das an den Träger gebundene oder das in der Lösung befindliche Konjugat nachgewiesen werden.

Für den Fall, daß der Analyt ein Antikörper ist, können die gleichen Prinzipien angewandt werden, jedoch muß dann anstelle der Antikörper in den oben beschriebenen Verfahren ein Antigen oder ein gegen den Antikörper gerichteter Antikörper verwendet werden.

In immunologischen Verfahren zum Nachweis eines Analyten schließt sich an den Nachweis des Hydrolasekonjugats nach dem erfindungsgemäßen Verfahren die Auswertung an. Aus der Anwesenheit oder bei quantitativer Auswertung der Menge des nachgewiesenen Hydrolasekonjugats kann nämlich auf die Anwesenheit bzw. die Menge des Analyten in der ursprünglich eingesetzten Probe geschlossen werden. Dies kann beispielsweise über eine Eichkurve geschehen.

Weiter kann das erfindungsgemäße Verfahren zum Nachweis einer Substanz mit Hydrolaseaktivität in Verfahren zum Nachweis von Nukleinsäuren eingesetzt werden. Solche Verfahren sind dem Fachmann auf dem Gebiet der Nukleinsäurehybridisierungstests bekannt. In diesen Tests werden einsträngige oder doppelsträngige Nukleinsäuren, insbesondere DNA oder RNA oder Fragmente davon, die mit einer Hydrolase als Enzymmarkierung verbunden sind, nachgewiesen, deren Menge ein Maß für die Konzentration der nachzuweisenden Nukleinsäure ist. Der Nachweis dieser hydrolasemarkierten Nukleinsäuren erfolgt in vorteilhafter Weise gemäß dem erfindungsgemäßen Verfahren zum Nachweis einer Substanz mit Hydrolaseaktivität.

Die Vorteile dieses neuen immunologischen Verfahrens und des neuen Verfahrens zum Nachweis von Nukleinsäuren ergeben sich aus den Vorteilen des erfindungsgemäßen Verfahrens zum Nachweis von Substanzen mit Hydrolaseaktivität.

Ein weiterer Gegenstand der Erfindung ist ein Mittel zum Nachweis von Substanzen mit Hydrolaseaktivität, welches mindestens eine Verbindung der Formel I enthält. Ein solches Mittel ist zum Nachweis von Substanzen mit Hydrolaseaktivität in einer Probe geeignet, der in einem vorangehenden, gleichzeitigen oder nachfolgenden Schritt ein Oxidationsmittel zugesetzt wird.

Bevorzugt ist ein Mittel zum Nachweis von Substanzen mit Hydrolaseaktivität, welches mindestens eine Verbindung der Formel I und ein Oxidationsmittel enthält.

Weiterhin kann das Mittel Zusatzstoffe enthalten, die zwar für die eigentliche Nachweisreaktion nicht benötigt werden, jedoch eine vorteilhafte Wirkung ausüben. Dazu gehören insbesondere pH-Puffersubstanzen, die es erlauben, die Nachweisreaktion bei einem konstanten pH-Wert auszuführen. Ebenfalls werden davon umfaßt Netzmittel, welche eine gleichmäßige Benetzung von Trägermaterialien bewirken, und Detergentien.

Das erfindungsgemäße Mittel enthält bevorzugt einen oder mehrere saug- oder quellfähigen Träger. Solche Träger sind beispielsweise Vliese oder Filme, bevorzugt Vliese. Geeignet sind auch schwammartige oder poröse Träger.

Dieses Mittel enthält bevorzugt einen oder mehrere saug- oder quellfähige Träger, auf welche das Hydrolasesubstrat und das Oxidationsmittel gemeinsam bzw. getrennt imprägniert sind oder im Fall von Filmen inkorporiert sind. Die Herstellung solcher Mittel erfolgt nach an sich bekannten Verfahren.

Das erfindungsgemäße Mittel kann beispielsweise außer dem Hydrolasesubstrat und dem Oxidationsmittel sowie eventuell vorhandenen Zusatzstoffen auch ein Lösungsmittel enthalten. Bevorzugtes Lösungsmittel ist Wasser. Auch hier können Hydrolasesubstrat und Oxidationsmittel gemeinsam oder getrennt als Lösung vorliegen.

Das erfindungsgemäße Mittel kann auch in Form eines oder mehrerer Pulver oder Pulvermischungen vorliegen. Außer dem Hydrolasesubstrat und dem Oxidationsmittel kann ein solches Mittel bevorzugt übliche inerte, lösliche galenische Füllstoffe, wie Polyvinylpyrrolidon, Polyethylenglykole usw. enthalten. Die Pulver oder Pulvermischungen können auch zu Tabletten gepreßt sein.

Ein solches Mittel ist dazu geeignet, in einem Verfahren zum Nachweis von Substanzen mit Hydrolase-Aktivität eingesetzt zu werden.

Ein weiterer Gegenstand der Erfindung ist die Verwendung von Verbindungen der Formel I in den oben geschilderten Verfahren zum Nachweis von Substanzen mit Hydrolaseaktivität in einer Probe.

Die Erfindung wird durch die folgenden Beispiele weiter erläutert:

## Beispiel 1:

**4-[(4-(4-Dimethylaminophenyl)-5-methyl)1-H-imidazol-2-yl]-2-methoxyphenol-hydrochlorid**

Eine Lösung von 30,4 g (0,2 Mol) 4-Hydroxy-3-methoxybenzaldehyd (Vanillin) in 400 ml Eisessig wird mit 38,24 g (0,2 Mol) 1-(4-Dimethylaminophenyl)propandion-1,2 und 154 g (2 Mol) Ammoniumacetat drei Stunden unter Rückfluß erhitzt und danach unter Eiskühlung in 1,1 l konz. Ammoniak eingetragen. Das erhaltene Rohprodukt wird in Essigester aufgenommen, mit Wasser durchgeschüttelt und das Lösungsmittel nach Trocknen über Natriumsulfat im Vakuum eingeengt. Nach Lösen des Rückstandes in Ethanol wird durch Zugabe von 5 N etherischer Salzsäure das Hydrochlorid gefällt und dieses durch Umkristallisieren aus Isopropanol-Wasser 3 : 1 gereinigt. Man erhält 58 g (81 %) farblose Kristalle der Titelverbindung. Fp. > 200 °C (Zers.) MS: m/e = 323 (Base).

Nach Oxidation der Leukoverbindung mit $H_2O_2$/POD --> Chromogen $\lambda_{max.}$ 693 nm, $\epsilon$ = 19500.

DC (Kieselgel 60, Essigester): 0,27

## Beispiel 2:

**4-[(4-(4-Dimethylaminophenyl)-5-methyl)oxazol-2-yl]-2-methoxyphenol**

a) **4-N,N-Dimethylamino-α-[(4-hydroxy-3-methoxy)-benzoyloxy]-propiophenon**
4,5 g (0,024 Mol) 4-Hydroxy-3-methoxybenzoesaures Natrium werden in 50 ml DMF suspendiert, 8 g (0,03 Mol) 4-N,N-Dimethylamino-α-brompropiophenon zugegeben und 6 Stunden unter Rückfluß erhitzt. Nach Einengen im Vakuum wird der Rückstand an einer Kieselgelsäule (Kieselgel 60) Ø 3 cm, Füllhöhe 26 cm chromatographiert (Laufmittel: n-Heptan/Methylethylketon 2/1). Man erhält 3,5 g (42,5 %) der Titelverbindung. Fp. 151/154 °C

b) **4-[(4-(4-Dimethylaminophenyl)-5-methyl)-oxazol-2-yl]-2-methoxyphenol**
Das unter 2a erhaltene Produkt wird mit 4,2 g (0,054 Mol) Ammoniumacetat in 18 ml Eisessig unter Argon 3 Stunden unter Rückfluß erhitzt, beim Abkühlen erhält man ein beigefarbenes Produkt, das nach Anrühren mit 50 ml Wasser, Absaugen und Trocknen über Diphosphorpentoxid 3,1 g (94,5 %) Titelverbindung, Fp. 170 - 173 °C, ergibt.

DC: (Kieselgel 60-F254, Laufmittel: Toluol/Methanol 5/1)
$R_F$ = 0,31, MS: m/e 324
$\lambda_{max}$ 542 nm, $\epsilon$ = 14558 nach Oxidation mit $H_2O_2$/POD

**Beispiel 3:**

**4-[(4-(4-Dimethylaminophenyl)-5-methyl)-thiazol-2-yl]-2,6-dimethoxyphenol**

a) **4-[4-(4-Dimethylaminophenyl)-5-methyl)-thiazol-2-yl]-2,6-dimethoxyphenol-benzylether**
7,4 g (0,024 Mol) 4-Benzyloxy-3,5-dimethoxy-phenylthioacetamid werden in 240 ml absolutem Ethanol gelöst, mit 6,2 g (0,026 Mol) 4-Dimethylamino-α-brompropiophenon versetzt und 6 Stunden unter Rückfluß erhitzt. Nach Absaugen und Waschen des Reaktionsproduktes mit abs. Ethanol erhält man 8,5 g (76 %) der Benzyloxyverbindung, gelbe Kristalle, Fp. 50 - 53 °C.
DC (Kieselgel 60-F254, Toluol/Essigester 5 : 1 - einheitlich)

b) **4-[(4-(4-Dimethylaminophenyl)-5-methyl)-thiazol-2-yl]-2,6-dimethoxyphenol**
Der in 3a erhaltene O-Benzylether wird in 300 ml Methanol unter Erwärmen gelöst und unter Kochen am Rückfluß 2 Stunden Chlorwasserstoffgas eingeleitet. Beim Abkühlen kristallieren 5,9 g (88,2 %), 3b, gelbe Kristalle.
Fp 230 - 233 °C.
MS : m/e = 370
$\lambda_{max}$ 564 nm . $\epsilon$ = 7687 nach Oxidation mit $H_2O_2$-POD.

**Beispiel 4:**

**4[(4-(4-Dimethylaminophenyl)-5-methyl)-1H-imidazol-2-yl]-2-methoxy-phenylacetat**

Man löst 3,5 g (0,01 Mol) 4[(4-(4-Dimethylaminophenyl)-5-methyl-1H-imidazol-2-yl]-2-methoxyphenol-hydrochlorid in 200 ml Pyridin, tropft 20 ml Acetanhydrid zu, rührt eine Stunde bei Raumtemperatur, saugt die gebildeten Kristalle ab, wäscht den Filterrückstand mit eiskaltem Ethanol und trocknet das erhaltene kristalline Acetat bei 40 °C im Trockenschrank. Man erhält 3,23 g (82 %) hellgelbe Kristalle der Titelverbindung. Fp 253 - 256 °C. MS: m/e 365
DC (Kieselgel 60 F 254 - Methylenchlorid/Methanol 5/1 - RF = 0,77)
Analog hergestellt:

**Beispiel 4a:**

**4-[(4-(4-Dimethylaminophenyl-5-methyl)-oxazol-2-yl]-2-methoxyphenylacetat**

MS: m/e = 366, Fp. 97/102 °C, Ausb. 75 %
DC (Kieselgel 60-F-254 - Toluol/Methanol 5/1) : RF = 0,36

**Beispiel 4 b:**

**4-[(4(4-Dimethylaminophenyl)-5-methyl)-thiazol-2-yl]-2,6-dimethoxy-phenylacetat**

MS: m/e = 412. Fp. 247 - 249 °C Ausbeute: 73%
DC (Kieselgel 60-F 254 - Toluol/Essigester 5/1) RF = 0,34

**Beispiel 4 c:**

**4-[(4-(4-Dimethylaminophenyl)-5-methyl)-1H-imidazol-2-yl]-2,6-dimethoxy-phenylacetat**

MS: m/e = 395, Fp. > 250 °C, Ausb. 78 %
DC ($SiO_2$ - 60 - F 254 - Toluol-Methanol 5 : 1, RF = 0,19 Enzymat. Spaltung und nachfolg. Oxidation mit $K_3[Fe(CN)_6]$ --> Chromogen: $\lambda_{max}$ 680 nm, 1g $\epsilon$ = 4,4

**Beispiel 4 d:**

**4-[4,5-Bis(4-dimethylaminophenyl)-1H-imidazol-2-yl]-2,6-dimethoxyphenylacetat**

MS: m/e = 500. Fp. 261 - 262 °C, Ausb. 72 %
DC (Kieselgel 60 - F 254 : Chloroform/Tetrahydrofuran 1/1): RF = 0,81

Enz. Spaltung und nachfolg. Oxidation mit $K_3[Fe(CN)_6$ --> Chromogen: $\lambda_{max}$ 643, 1g $\epsilon$ = 4,36

**Beispiel 5:**

**4-[(4-(4-Dimethylaminophenyl)-5-methyl)-1H-imidazol-2-yl]-2-methoxyphenyl-dodecanoat**

Man löst 1,6 g (0,005 Mol) 4-[4-(4-Dimethylaminophenyl)-5-methyl)-1-H-imidazol-2-yl]-2-methoxy-phenol in 20 ml abs. Pyridin und tropft innerhalb 10 Minuten 3,6 ml (0,01 Mol) Dodecansäurechlorid 98 % unter Rühren zu. Nach einstündigem Weiterrühren gibt man 50 ml Eiswasser zu und extrahiert zweimal mit je 50 ml Essigester. Nach Trocknen über Natriumsulfat und Abdampfen des Lösungsmittels wird das Rohprodukt säulenchromatographisch gereinigt ($SiO_2$, Toluol/Essigester 1 : 1). Man erhält 1,3 g (48 %) beigefarbene Kristalle der Titelverbindung.

MS: m/e = 505. Fp. 110 - 112 °C

DC: (Kieselgel 60 - F254 - Toluol/Methanol 5/1) RF = 0,34,

Analog hergestellt:

**Beispiel 5 a:**

**4-[(4(4-Dimethylaminophenyl)-5-methyl)-1-H-imidazol-2-yl]-2-methoxyphenyl-propanoat**

MS: m/e = 379, Fp. 244 - 246 °C, Ausb. 56 %

DC (Kieselgel 60 - F 254 - Toluol/Essigester 1/1) RF = 0,31

**Beispiel 5 b:**

**4-[(4-(4-Dimethylaminophenyl)-5-methyl)-1H-imidazol-2-yl]-2-methoxyphenyl-butanoat**

MS: m/e = 393, Fp. 62 - 64 °C, Ausb. 63 %

DC (Kieselgel 60 F 254 - Toluol/Essigester 1/1, RF = 0,33

**Beispiel 5 c:**

**4-[(4(4-Dimethylaminophenyl)-5-methyl)-1-H-imidazol-2-yl]-2-methoxyphenyl-pentanoat**

MS: m/e = 407, amorph, Ausb. 72 %

DC (Kieselgel 60 - F 254 - Toluol/Essigester 2/1, RF = 0,18.

**Beispiel 5 d:**

**4-[(4-(4-Dimethylaminophenyl)-5-methyl)-1-H-imidazol-2-yl]-2-methoxyphenyl-octanoat**

MS: m/e = 449. Fp. 126 - 129 °C, Ausb. 78 %

DC (Kieselgel 60 - F 254 - Toluol Essigester 1/1): RF = 0,44

**Beispiel 6:**

**4-[(4-(4-Dimethylaminophenyl)-5-methyl)-1-H-imidazol-2-yl]-2-methoxyphenyl-dihydrogenphosphat**

Zu einer Mischung von 20 ml Pyridin und 6 ml (0,065 Mol) Phosphoroxytrichlorid tropft man innerhalb von 10 Minuten eine Lösung von 3,2 g (0,01 Mol) 4-[(4-(4-Dimethylaminophenyl)-5-methyl)-1-H-imidazol-2-yl]-2-methoxy-phenol in 20 ml Pyridin, wobei die Temperatur auf ca. 30 °C ansteigt. Man rührt drei Stunden bei Raumtemperatur und saugt nach zwölfstündigem Stehen bei RT die gebildeten Kristalle ab, wäscht mit Wasser und trocknet das Rohprodukt über Diphosphorpentoxid. Die säulenchromatographische Reinigung an Kieselgel 60 (Säule : Füllhöhe 84 cm Ø 2,5 cm) mit Isopropanol/n-Butylacetat/Wasser/Ammoniak 50 : 30 : 15 : 5 gibt 1,4 g (24 %) der Titelverbindung.

MS: m/e = 403, Fp. 226 /230 °C

DC (Kieselgel 60 - F 254 - n -

Propanol/Essigester/$H_2O$/Essigsäure

60 : 10 : 30 : 5, RF = 0,51
Analog hergestellt:

**Beispiel 6 a:**

**4-[(4-(4-Dimethylaminophenyl)-5-methyl)-1-H-imidazol-2-yl]-2,6-dimethoxyphenyl-dihydrogenphosphat**

MS: m/e = 433, Fp. 196 - 198 °C, Ausb. 35 %
DC (Kieselgel 60 - F 254, Methylenchlorid/Methanol 75/75 ) einheitlich.

**Beispiel 6 b:**

**4-[(4-(4-Dimethylaminophenyl)-5-phenyl)-1-H-imidazol-2-yl]-2,6-dimethoxyphenyl-dihydrogenphosphat**

MS: m/e = 495, Fp. 230/235 °C, Ausb. 28 %
erhalten aus:
4[(4-(4-Dimethylaminophenyl-5-phenyl)-1-H-imidazolyl)-2,6-dimethoxyphenol aus dem entsprechenden Diketon analog Beisp.1 MS: m/e 410, Fp. 181 °C (Zers.) $\lambda_{max}$ nach Oxidation mit $H_2O_2$/POD 680 nm . 1g $\epsilon$ = 4,3

**Beispiel 6 c:**

**4-[4,5-Bis(4-dimethylaminophenyl)-1-H-imidazol-2-yl]-2,6-dimethoxyphenyl-dihydrogenphosphat**

MS: m/e = 538, Fp. 258 - 260 °C, Ausb. 45 %
aus 4[(4,5-bis(4-Dimethylaminophenyl)-1-H-imidazol-2-yl]-2,6-dimethoxyphenol (KODAK)
$\lambda_{max}$ nach Oxidation mit $H_2O_2$/POD 643 nm, 1g $\epsilon$ = 4,4

**Beispiel 7:**

**4-[(4(4-Dimethylaminophenyl)-5-methyl)-oxazol-2-yl]-2-methoxyphenyl-dihydrogenphosphat**

a) **4-[(4(4-Dimethylaminophenyl-5-methyl)-oxazol-2-yl]-2-methoxyphenyldibenzyloxyphosphat**
Man suspendiert 3,24 g (0,01 Mol) 4-[ (4(4-Dimethylaminophenyl-5-methyl)-oxazol-2-yl]-2-methoxyphenol in 40 ml abs. Tetrahydrofuran, gibt 2 ml Tetrachlorkohlenstoff zu und erwärmt im Ölbad auf 40 °C, wobei das Oxazol weitgehend in Lösung geht. Danach tropft man nacheinander 6 ml Dibenzylphosphat und 4 ml Triethylamin innerhalb fünf Minuten zu. Man rührt fünf Stunden bei Raumtemperatur, saugt das gebildete Triethylammoniumchlorid ab und engt das Filtrat im Vakuum ein. Das erhaltene Rohprodukt wird säulenchromatographisch gereinigt. (Kieselgel 60 Säule 1,1 m, Ø 3 cm - Toluol/Methanol 20/1).
b) **4-[(4(4-Dimethylaminophenyl)-5-methyl)-oxazol-2-yl]-2-methoxyphenyl-dihydrogenphosphat**
1 g der Verbindung aus a) werden in einer Mischung von 8 ml Methanol und 8 ml Dioxan unter Zugabe von 0,06 g PdO viereinhalb Stunden bei RT hydriert. Danach reinigt man das Endprodukt säulenchromatographisch und erhält 0,8 g (19,8 %) der Titelverbindung, gelbes Öl.
MS: m/e = 404, amorph DC (Kieselgel 60 F 254 - n-Propanol/Essigester/Wasser/Toluol/Eisessig
60/10/30/5/2): RF = 0,74

**Beispiel 7 c:**

**4[(4,5-bis(4-Dimethylaminophenyl)oxazol-2-yl]-2,6-dimethoxyphenyl-dihydrogenphosphat**

MS: m/e = 539. Fp. 195/200 °C
aus 4[4,5-bis-(4-Dimethylaminophenyl)oxazol-2-yl]-2,6-dimethyloxyphenol.
MS: m/e = 459, Fp. 218 °C
$\lambda_{max}$ nach Oxidation mit $H_2O_2$/POD 555 nm, 1g $\epsilon$ = 3,85

**Beispiel 8:**

**(4-(4-(4-Dimethylaminophenyl)-5-methyl)-1H-imidazol-2-yl)-2-methoxyphenyl)-2-(acetamido)-2-deoxy-$\beta$-D-glucopyranosid**

a) **2[4-(2-Acetamido-2-deoxy-3,4,6-triacetyl-$\beta$-D-glucopyranosyloxy)-3-methoxyphenyl]-5-methyl)-4(4-dimethylaminophenyl)-1,3-imidazol (MS = 651)**

Zu einer Lösung von 7 g (0,025 Mol) n-Butyltriethylammoniumbromid in 200 ml Wasser gibt man eine Lösung von 5,9 g (0,0164 Mol) 4[(4-(4-Dimethylaminophenyl)-5-methyl)-1-H-imidazol-2-yl]-2-methoxyphenol in 200 ml Chloroform, ferner 14,2 g (0,039 Mol) 1-Chlor-2-desoxy-2,3,4,6-tetraacetyl-$\beta$-D-glucosamin und 13,9 g (0,102 Mol) Kaliumcarbonat. Unter kräftigem Rühren kocht man vier Stunden unter Rückfluß. Nach Abkühlen trennt man die Chloroformphase ab und engt das Lösungsmittel im Vakuum ein. Das erhaltene Rohprodukt wird säulenchromatographisch an Kieselgel gereinigt. (Laufmittel-Methylenchlorid/Methanol 1 : 1). Man erhält 4,4 g (41,1 %) der Tetraacetylverbindung.

b) **(4-(4-(4-Dimethylaminophenyl)-5-methyl)-1H-imidazol-2-yl)-2-methoxy-phenyl)-2-acetamido-2-deoxy-$\beta$-D-glucopyranosid**

Zur Deacetylierung des in 8a) erhaltenen Produktes wird dieses in 300 ml Methanol gelöst und nach Zugabe von 20 g Natriumhydrogencarbonat 3 Stunden bei 30 °C gerührt. Nach Absaugen des anorganischen Materials wird das Lösungsmittel eingeengt und das Endprodukt säulenchromatographisch an Kieselgel 60 Laufmittel: Toluol/Methanol 1 : 1 nachgereinigt. Es resultieren 3,1 g (36 %) DC-einheitliches Produkt, das nach Anrühren mit eiskaltem Methanol 1,8 g (21 %) der Titelverbindung ergibt.

MS: m/e 525, Fp. 181 °C

DC (Kieselgel 60 F 254, Toluol/Essigester/Methanol 1 : 1 : 1)

RF = 0,21

Die Verbindung stellt bei pH 6 und pH 9 ein sehr gutes Substrat für N-Acetyl-$\beta$-D-glucosaminidase dar (starke Blaufärbung).

**Beispiel 9:**

**Herstellung von 2-(4-$\beta$-D-galactopyranosyloxy-3-methoxyphenyl)-5-methyl-4-(4-dimethylaminophenyl)-1,3-imidazol**

a) **2-[4-(Tetra-O-acetyl-$\beta$-D-galactopyranosyloxy)-3-methoxyphenyl]-5-methyl-4-(4-dimethylaminophenyl)-1,3-imidazol**

Eine Lösung von (4-Formyl-2-methoxyphenyl)2,3,4,6-tetra-O-acetyl-$\beta$-D-galactopyranosid [R. G. Price et al., Clin. Chim. Acta 124 (1982) 195-204; 4,8 g, 10 mMol] 1-(4-Dimethylaminophenyl)propandion-1,2(1,9 g, 10 mMol) und Ammoniumacetat (7,7 g, 100 mMol) in Eisessig (200 ml) wird 1 Stunde auf 125 °C erhitzt. Anschließend wird auf Eis/Wasser gegossen, der Niederschlag abgesaugt und getrocknet. Nach säulenchromatographischer Aufreinigung ($SiO_2$; Toluol/Essigester/Methanol = 4:4 : 1) erhält man 4,2 g (65 %) der Titelverbindung vom Fp. 163-168 °C, m/e 653 ($M^+$).

b) **2-(4-$\beta$-D-galactopyranosyloxy-3-methoxyphenyl)-5-methyl-4-(4-dimethylaminophenyl)1,3-imidazol**

Die unter a erhaltene Verbindung (5 g, 7,6 mMol) in Methanol (30 ml) wird bei 0 °C mit 1 N Na-methylat-Lösung (5 ml) versetzt. Nach 3 Stunden wird die Lösung mit einem schwach sauren Ionenaustauscher (z. B. Amberlite® IRC 50, $H^+$-Form) behandelt, abfiltriert und eingeengt. Der Rückstand wird mit Ether/Petrolether digeriert und abfiltriert; Ausbeute 2,7 g (73 %); Fp. 140 °C (unter Aufschäumen).

MS (neg. FAB): m/e = 484

DC (Kieselgel 60, Isopropanol/Methanol 1/1): RF = 0,4.

**Beispiel 10:**

**Herstellung von 2-(4-$\beta$-D-galactopyranosyloxy-3-methoxyphenyl)-4,5-bis(4-dimethylaminophenyl)-1,3-imidazol**

a) In gleicher Weise wie in Beispiel 9 wurde (4-Formyl-2-methoxyphenyl)-2,3,4,6-tetra-O-acetyl-$\beta$-D-galactopyranosid [R. G. Price et al., Clin. Chim. Acta 124 (1982) 195-204; 4,8 g, 10 mMol], 4,4′-Bis-(dimethylamino)benzil (3 g, 10 mMol) und Ammoniumacetat (7,7 g, 100 mMol) in Eisessig (200 ml) umgesetzt. Nach 18stündigem Kochen unter Rückfluß, Aufarbeitung und chromatographischer Aufreinigung wurde das Tetra-O-acetylgalactosyl-Derivat erhalten; Ausbeute 4,7 g (62 %).

b) Zur Deacetylierung wurde das zuvor erhaltene Produkt (1,26 g, 1,7 mMol) mit einer Suspension von Natriumhydrogencarbonat (1,7 g) in Methanol (150 ml) zwei Stunden lang bei 40 °C behandelt. Nach Abfiltrieren des anorganischen Niederschlags wurde eingeengt und der Rückstand chromatographisch aufgereinigt (Kieselgel 60, Toluol/Essigester/Methanol = 2 : 2 : 1); Ausbeute 0,77 g (77 %); Fp. 174-182 °C.

**Beispiel 11:**

In analoger Weise wurden in zwei Reaktionsschritten, wie in Beispiel 10 beschrieben, die nachstehend aufgeführten Imidazol-Derivate erhalten. Im ersten Schritt wurden aus den entsprechenden peracetylierten (4-Formylphenyl)-β-D-galactosiden und den entsprechenden 1,2-Diketonen durch Umsatz mit Ammoniumacetat in Eisessig die peracetylierten Galactoside hergestellt, die im zweiten Reaktionsschritt deacetyliert wurden.

a) **2-(4-β-D-galactopyranosyloxyphenyl)-4-(4-dimethylaminophenyl)-5-methyl-1,3-imidazol**
Aus (4-Formylphenyl)2,3,4,6-tetra-O-acetyl-β-D-galactopyranosid (Z. Csuros et al., Acta Chim. Acad. Sci. Hung, 42 (1964) 263-267), 4,5 g, 10 mMol
und
1-(4-Dimethylaminophenyl)propandion-1,2 (1,9 g, 10 mMol);
Reaktionsdauer: 2 h;
Tetra-O-acetyl-β-D-galactopyranosid: Ausbeute: 4,6 g (74 %).
Deacetylierung analog Beispiel 10b; Ausbeute 74 %,
Fp. 177-187 °C.

b) **2-(4-β-D-galactopyranosyloxyphenyl)-4,5-bis(4-dimethylaminophenyl)-1,3-imidazol**
Aus (4-Formylphenyl)-2,3,4,6-tetra-O-acetyl-β-D-galactopyranosid [Z. Csuros et al., Acta Chim. Acad. Sci. Hung., 42 (1964) 263-267]; 4,5 g, 10 mMol,
und
4,4'-Bis(dimethylamino)benzil (3 g, 10 mMol)
Reaktionsdauer: 2 h;
Tetra-O-acetyl-β-D-galactopyranosid: Ausbeute: 2,2 g (30 %).
Deacetylierung analog Beispiel 10b; Ausbeute 83 %, Fp. 230-237 °C

c) **2-(3-Chlor-4-β-D-galactopyranosyloxyphenyl)-5-methyl-4-(4-dimethylaminophenyl)-1,3-imidazol**
Aus (2-Chlor-4-formylphenyl)2,3,4,6-tetra-O-acetyl-β-D-galactopyranosid [hergestellt aus 2,3,4,6-Tetra-O-acetyl-α-D-galactopyranosylbromid und 2-Chlor-4-formylphenol entsprechend der Vorschrift von R. G. Price et al., Clin. Chim. Acta 124 (1982) 195-204]; 4,9 g, 10 mMol,
und
1-(4-Dimethylaminophenyl)propandion-1,2(1,9 g, 10 mMol);
Reaktionsdauer: 2 h,
Tetra-O-acetyl-β-D-galactopyranosid; Ausbeute: 5,95 g (90 %).
Deacetylierung analog Beispiel 10b.

d) **2-(3-Chlor-4-β-D-galactopyranosyloxyphenyl)-4,5-bis(4-dimethylaminophenyl)-1,3-imidazol**
Aus (2-Chlor-4-formylphenyl)2,3,4,6-tetra-O-acetyl-β-D-galactopyranosid [hergestellt aus 2,3,4,6,-Tetra-O-acetyl-α-D-galactopyranosylbromid und 2-Chlor-4-formylphenol entsprechend der Vorschrift von R. G. Price et al., Clin. Chim. Acta 124 (1982) 195-204]; 4,4 g, 9 mMol
und
4,4'-Bis(dimethylamino)benzil (2,7 g, 9 mMol)
Reaktionsdauer: 6 h;
Tetra-O-acetyl-β-D-galactopyranosid: Ausbeute: 6,9 g Rohprodukt, das ohne chromatographische Reinigung der Deacetylierung analog Beispiel 10b unterworfen wurde.

**Beispiel 12:**

**2-(4-β-D-galaktopyranosyloxy-3-methoxyphenyl)-4,5-bis(4-methoxy-phenyl)-1,3-imidazol**

a) **2-(4-(tetra-O-acetyl-β-D-galaktopyranosyloxy)-3-methoxyphenyl]-4,5-bis(4-methoxyphenyl)-1,3-imidazol**
Eine Lösung aus 14 g (29 mMol) Vanillin-2,3,4,6-tetra-o-acetyl-β-D-galactopyranosid (Price et al., a.a.o.), 7,8 g (29 mMol) 4,4'-Dimethoxybenzil und 13,2 g (174 mMol) Ammoniumacetat in 700 ml Eisessig wird

unter Rühren und in Schutzgasatmosphäre 5 h unter Rückfluß gekocht. Anschließend läßt man auf 20 °C abkühlen und dampft die Essigsäure unter mehrfacher Zugabe von Toluol im Vakuum ab. Das Rohprodukt wird ohne weitere Aufreinigung in der nächsten Synthesestufe eingesetzt. DC (Kieselgel 60, Laufmittel Ethylacetat/Petrolether 1/1): RF = 0,1.

b) **2-(4-β-D-galaktopyranosyloxy-3-methoxyphenyl)-4,5-bis(4-methoxy-phenyl)-1,3-imidazol**

Das unter a) erhaltene Rohprodukt wird in 300 ml absolutem Methanol suspendiert und mit einer ausreichenden Menge einer gesättigten methanolischen Natriummethylatlösung versetzt, um einen pH-Wert von 12 - 13 zu erreichen. Man läßt bei 20 °C ca 1 h bis zur vollständigen Deacetylierung rühren (DC-Kontrolle, pH-Kontrolle) und neutralisiert die Lösung durch Zugabe von saurem Ionenaustauscher (Dowex® 50 WX 8, $H^+$). Nach 15 min wird der Ionenaustauscher abfiltriert und das Filtrat im Vakuum eingeengt. Die Aufreinigung erfolgt durch Flash-Chromatographie an Kieselgel (Eluens: Ethylacetat/Methanol 7/3). DC (Kieselgel 60, Laufmittel Ethylacetat/Methanol 7/3):
RF = 0.4. Ausbeute: 4,9 g (30 %). MS (neg. FAB):
m/e = 563.

## Beispiel 13:

**2-(4-β-D-galaktopyranosyloxy-3-methoxyphenyl)-4,5-bis(4-methylphenyl)-1,3-imidazol**

Mit 4,4′-Dimethylbenzil wurde analog zu Beispiel 12 das entsprechende Galactosid hergestellt. DC (Kieselgel 60, Laufmittel Ethylacetat/Methanol 7/3): RF = 0,5. MS (neg. FAB): m/e = 531.

## Beispiel 14:

### Eignung als Hydrolase-Substrate

Untersucht wurde in einem Tüpfeltest die Hydrolyse der verschiedenen Verbindungen durch entsprechende Hydrolasen (Arylsulfatase, saure und alkalische Phosphatase, Esterase, Acetyl-β-O-glucosamidase und Lipase).

Die dabei freiwerdende Base wurde gleichzeitig durch Kaliumhexacyanoferrat (III), $K_3[Fe(CN)_6]$, zum Farbstoff oxidiert und die Farbbildung visuell bewertet.

### Durchführung/Ergebnisse

Die Hydrolysegeschwindigkeit wurde bei pH 6,0 sowie bei pH 9,0 durch Tüpfeln einer Hydrolase-Lösung auf ein Substrat-Puffer-Oxidationsmittel-Papier visuell bewertet.

Die Enzymkonzentration betrug ca. 1000 U/ml, die Prüftemperatur war dabei Raumtemperatur.

Vergleichend wurde die nichtenzymatische Hydrolyse mit 0,9%iger NaCl-Lösung als Probe bewertet.

Durch Einsatz der entsprechenden freien Base wurde die oxidierbarkeit mit $K_3[Fe(CN)_6]$ belegt.

Herstellung der Substrat-Papiere

| | | |
|---|---|---|
| Vlies pH 6,0-Tränklösung | 100 mMol/l | Kaliumdihydrogenphosphat |
| | 10 mMol/l | Kaliumhexacyanoferrat III |
| | 2 % | Pluronic ®F 68 |

Der pH-Wert wurde mit 2N KOH-Lösung auf 6,0 eingestellt.

| | | |
|---|---|---|
| Vlies pH 9,0- | 100 mMol/l | Glycin |
| | 10 mMol/l | Kaliumhexacyanoferrat III |
| | 2 % | Pluronic ®F 68 |

Der pH-Wert wurde mit 2N NaOH-Lösung auf 9,0 eingestellt.

Tränkung: Ein 8 x 30 cm Papiervlies (Whatman 3M) wurde mit 8 ml Tränklösung getränkt und 30 Minuten im Trockenschrank bei 50 °C getrocknet.

Die so imprägnierten Puffer-Oxidationsmittel-Papiere wurden in 8 x 2 cm Streifen geschnitten und mit Substratlösung nachimprägniert.

Substrat-Lösung/
Tränklösung: In 1 ml Methanol wurden 5 mg zu testendes Substrat (bzw. freie Base) gelöst oder suspendiert und davon je 10 µl auf die Puffer-Oxidationsmittel-Papiere dosiert. Mit dem Föhn wurde das Lösungsmittel abgedampft.

Auf diese Substrat-Oxidationsfläche wurden dann die Hydrolase-Lösungen dosiert und bewertet. Die in Beispiel 1 bis 7a beschriebenen Verbindungen zeigten auf den obigen Papieren positive Ergebnisse:

| Beispiel | Enzym | pH 6 | pH 9 |
|---|---|---|---|
| 4 | 1 | + + | + + + |
| 4a | 1 | + + + | + + + |
| 4b | 1 | + | + + |
| 4c | 1 | + + + | + + + |
| 4d | 1 | + + | + + |
| 5 | 1 | + | + + |
| 5a | 1 | + | + + |
| 5b | 1 oder 4 | + + | + + + |
| 5c | 1 | + + | + + |
| 5d | 1 | + + | + + + |
| 6 | 2 | - | + + |
| 6a | 2 | - | + + + |
| 6b | 2 | + + | + + + |
| 6c | 2 | + | + + |
| 7b | 2 | + | + + |
| 7c | 2 | + + | + + + |
| 8 | 3 | + + + | + + + |

Enzyme
1 Esterase aus Schweineleber
2 Alkalische Phosphatase
3 N-Acetyl-β-D-glucosaminidase
4 Lipase

Färbung
+ Färbung
+ + starke Färbung
+ + + starke und rasche Färbung

**Beispiel 15**

**Nachweis von menschlichem Choriongonadotropin (hCG) in einer Probe**

**A) Herstellung eines Testträgers 1 (Fig. 1)**

**a) Herstellung von Vlies 3**

Ein Stück Papier (Typ 4210 der Firma Kalff) wurde in einen Streifen (2,6 cm lang und 0,6 cm breit) geschnitten. 20 µl einer Losung von 40 mg/ml 2-Methoxy-4-[4,5-bis-(4-methoxyphenyl)-imidazol-2-yl]-phenyl-β-D-galactopyranosid (Galactosidasesubstrat aus Beispiel 12) in DMSO wurden mit 20 µl einer 24 %igen Lösung von Polyvinylalkohol in Wasser vermischt. Diese Mischung wurde auf die Mitte des Papierstreifens aufgegeben.

**b) Herstellung von Vlies 5**

Ein Stück Papier (Typ 4210 der Firma Kalff) wurde in einen Streifen (2,6 cm lang und 0,6 cm breit) geschnitten. Ein Ende wurde mit 100 µl PBS-Puffer (Phosphate buffered saline, pH 7,0, 1 % Rinderserum Albumin, 0,1 % Tween$^{R}$20) imprägniert. Auf die Mitte des Streifens wurden 7,5 µl einer Lösung aufgegeben, die 20 U/ml eines Konjugats aus einem Fab-Fragment eines monoklonalen Antikörpers gegen hCG und β-Galactosidase (Substanz mit Galactosidaseaktivität), 100 µg/ml eines monoklonalen Maus-Antikörpers gegen die β-Kette von hCG und 7,5 mg/ml 4-Aminobenzyl-1-thio-β-D-galactopyranosid enthält. Das Ende, welches dem pufferimprägnierten Ende gegenüberliegt, wurde mit 10 µl einer 5 %igen Lösung von Polyvinylalkohol in Wasser imprägniert.

**c) Herstellung von Vlies 6**

An ein Stück Papier (Typ 3512 der Firma Schleicher & Schüll) wurde nach Aktivierung mit Bromcyan Schafantikörper gegen den Fc-Teil von Mausantikörpern fixiert. Ein Stück dieses Materials wurde in einen Streifen (Länge 1,1 cm, Breite 0,6 cm) geschnitten.

d) Herstellung von Vlies 7
Ein Stück Papier (Typ D-28 der Firma Whatman) wurde auf die Größe von 5 * 0,6 cm geschnitten.
**e)** Vlies 3 und 5 sind durch eine Plastikfolie 4 voneinander getrennt.

Durch Aufkleben der getrockneten Vliese auf eine Grundfolie 2 mit der Breite 0.6 cm wurde der in Figur 1 dargestellte Testträger 1 hergestellt. Vlies 5 wird dabei so aufgeklebt, daß das mit Polyvinylalkohol imprägnierte Ende in Richtung des Endes 8 des Testträgers zeigt.

**B) Durchführung des Tests**

**a) Probenvorbereitung**
0,5 ml Probe wurden mit 0,5 ml einer Lösung, welche 4 mmol/l Natriumperborat und 10 mg/l Meerrettich-Peroxidase (Oxidationsmittel) in Phosphatpuffer pH 7 enthält, gemischt.
**b) Aufgabe der Probe auf den Teststreifen**
Das Ende 8 des Teststreifens 1 wurde in die Lösung gestellt. Die hCG enthaltende Lösung drang in Vlies 5 und Vlies 3 ein und löste die darauf befindlichen Substanzen auf. Durch Reaktion von hCG mit dem β-galactosidasemarkierten Fab-Fragment eines monoklonalen Antikörpers gegen hCG und dem monoklonalen Antikörper gegen die β-Kette von hCG bildete sich in der Lösung in Vlies 5 ein β-galactosidasemarkierter Immunkomplex aus den drei Komponenten. Dieser Immunkomplex, der in diesem Fall die Substanz mit Hydrolaseaktivität darstellt, wird nun mittels des erfindungsgemäßen Verfahrens in Vlies 6 nachgewiesen. Die Lösung mit dem Immunkomplex drang genauso wie die Lösung mit dem Substrat aus Vlies 3 in Vlies 6 ein und wurde dort mit dieser Substratlösung gemischt. In Vlies 6 wurde der gebildete Immunkomplex sowie der Überschuß des monoklonalen Antikörpers gegen die β-Kette von hCG über die fixierten Schafantikörper an Vlies 6 gebunden. Ein eventueller Überschuß an β-galactosidasemarkiertem Fab-Fragment floß weiter in Vlies 7. Durch die β-Galactosidasemarkierung des gebundenen Immunkomplexes und mit Hilfe des Oxidationsmittels bildete sich innerhalb von 10 Minuten eine rote Farbe in Vlies 6.
Ist kein hCG in der Probe enthalten gewesen, so konnte sich auch kein β-galactosidasemarkierter Immunkomplex bilden. Daher wird keine Substanz mit Hydrolaseaktivität in Vlies 6 gebunden und somit keine Farbentwicklung gefunden. Hingegen ist die gesamte Menge an β-galactosidasemarkiertem Fab-Fragment noch in der Lösung enthalten, welche in Vlies 7 eindringt. Man findet daher in Vlies 7 eine Farbentwicklung, die von der Reaktion des β-galactosidasemarkierten Fab-Fragments herrührt, welches auch eine Substanz mit Hydrolaseaktivität ist. Somit findet eine Kontrolle des Tests dadurch statt, das auch in Vlies 7 das erfindungsgemäße Verfahren durchgeführt wird; dies ist besonders in dem Fall wichtig, wenn kein hCG in der Probe vorhanden war.

Die Auswertung kann auch quantitativ erfolgen, denn je mehr hCG in der Probe und somit gebundener β-galactosidasemarkierter Immunkomplex in Vlies 6 vorliegt, desto intensiver wird die Färbung in Vlies 6 nach Ablauf der 10 Minuten sein.

**Beispiel 16**

**Nachweis von Thyroxin (T4)**

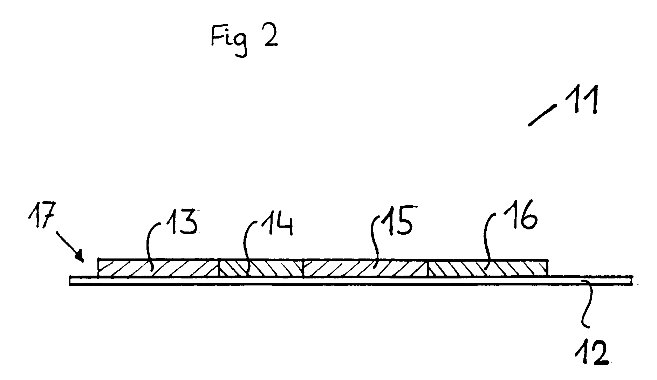

**A) Herstellung eines Teststreifens 11 (Fig. 2)**

**a) Vlies 14**
Vlies 14 ist mit einem Konjugat von β-Galactosidase mit einem Antikörper gegen T4 imprägniert.
**b) Vlies 15**
Ein Stück Papier (Schleicher & Schüll 3512) wurde mit Bromcyan aktiviert und daran T4 Succinimidester gebunden.
**c) Vlies 16**
Vlies 16 ist ein Vlies, welches mit 2-Methoxy-4-[4,5-bis-(4-methoxyphenyl)-imidazol-2-yl]-phenyl-β-D-galactopyranosid (Beispiel 12) imprägniert wurde.
**d)** Vlies 13 dient zur Probenaufgabe.
Zur Herstellung des Testträgers 11 wurden die Vliese, wie in Figur 2 gezeigt, auf einer Grundfolie 12 befestigt.

B) Durchführung des Tests

a) Probenvorbereitung
Die Probe wurde analog zu Beispiel 15 vorbereitet.
b) Durchführung
Der Teststreifen 11 wurde mit dem Ende 17 in die Lösung gestellt. Die T4-haltige Probe drang in Vlies 13 ein. Von dort floss sie weiter in Vlies 14, von wo sie das $\beta$-Galactosidase-Konjugat ablöste. Durch Immunreaktion des Antikörpers mit T4 bildete sich ein $\beta$-galactosidasemarkierter Immunkomplex. Die Lösung, welche nun neben diesen Immunkomplex noch einen Überschuß des $\beta$-Galactosidasekonjugats enthielt, drang in Vlies 15 ein. Dort wurde dieser Überschuß über gebundenes T4 an das Papier gebunden.

Die Lösung, welche noch den Immunkomplex enthält, drang in Vlies 16 ein. Dort findet das erfindungsgemäße Verfahren zum Nachweis des $\beta$-galactosidasemarkierten Immunkomplexes, welcher die Substanz mit Hydrolaseaktivität darstellt, statt. Je mehr dieses Komplexes und somit auch von T4 in der Lösung enthalten war, desto stärker hatte sich Vlies 16 nach 10 Minuten rot gefärbt. Bei Abwesenheit von T4 blieb Vlies 16 weiß.

## Patentansprüche

**1.** Verbindung der Formel I

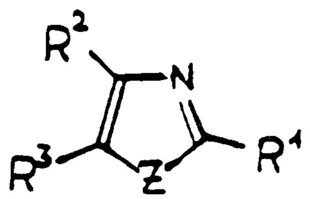

(I)

in der

Z Sauerstoff, Schwefel oder die Gruppe N-$R^4$ bedeutet

$R^1$, $R^2$ und $R^3$ gleich oder verschieden sein können und Wasserstoff, eine Alkyl- oder Aralkylgruppe oder einen aromatischen Rest bedeuten

$R^4$ Wasserstoff oder eine Alkylgruppe darstellt

und in der ein oder mehrere der Reste $R^1$, $R^2$ und $R^3$ einen durch die Gruppe

-O-X

in der

O Sauerstoff und

X einen Glykosyl-, Phosphat- oder Acylrest vorstellen, substituierten aromatischen Rest bedeuten.

**2.** Mittel zum Nachweis von Substanzen mit Hydrolaseaktivität in einer Probe, dadurch gekennzeichnet, daß es mindestens eine Verbindung gemäß Anspruch 1 enthält.

**3.** Mittel nach Anspruch 2, dadurch gekennzeichnet, daß es zusätzlich ein Oxidationsmittel enthält.

**4.** Verfahren zum Nachweis von Substanzen mit Hydrolase-Aktivität in einer Probe durch Mischen der Probe mit einem Hydrolasesubstrat und einem Oxidationsmittel und Auswertung der entstehenden Farbintensität, dadurch gekennzeichnet, daß als Hydrolasesubstrat mindestens eine Verbindung gemäß Anspruch 1 verwendet wird.

**5.** Verfahren nach Anspruch 4, dadurch gekennzeichnet, daß die Auswertung visuell erfolgt.

**6.** Verfahren zur Herstellung von Verbindungen gemäß Anspruch 1, dadurch gekennzeichnet, daß Verbindungen der Formel II

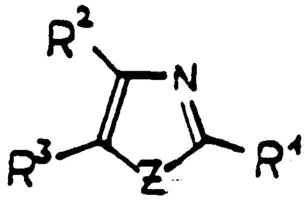

(II),

in der

| | |
|---|---|
| Z | Sauerstoff, Schwefel oder die Gruppe N-$R^4$ bedeutet |
| $R^1$, $R^2$ und $R^3$ | gleich oder verschieden sein können und Wasserstoff, eine Alkyl- oder Aralkylgruppe oder einen aromatischen Rest bedeuten |
| $R^4$ | Wasserstoff oder eine Alkylgruppe darstellt |

und in der ein oder mehrere der Reste $R^1$, $R^2$ und $R^3$ einen durch die Gruppe

-O-H

in der

O Sauerstoff vorstellt,

substituierten aromatischen Rest bedeuten,
mit Verbindungen der Formel III,

X-Y (III),

in der X die in Anspruch angegebene Bedeutung hat und Y eine reaktive Gruppe bedeutet, umgesetzt werden.

7. Verwendung von Verbindungen gemäß Anspruch 1 zum Nachweis von Substanzen mit Hydrolaseaktivität.

8. Teststreifen zum Nachweis eines Analyten unter Verwendung einer hydrolasemarkierten Verbindung und eines Hydrolasesubstrates dadurch gekennzeichnet, das als Hydrolasesubstrat eine Verbindung der Formel I eingesetzt wird, welches auf einen saugfähigen Träger imprägniert ist.

## Claims

1. Compounds of the formula I

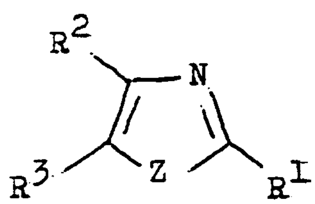

(I)

in which Z signifies oxygen, sulphur or the group N-$R^4$, $R^1$, $R^2$ and $R^3$ can be the same or different and signify hydrogen, an alkyl or aralkyl group or an aromatic radical, $R^4$ represents hydrogen or an alkyl group and in which one or more of the radicals $R^1$, $R^2$ and $R^3$ signifies an aromatic radical substituted by the group -O-X, in which O represents oxygen and X a glycosyl, phosphate or acyl radical.

2. Agent for the detection of substances with hydrolase activity in a sample, characterised in that it contains at least one compound according to claim 1.

3. Agent according to claim 2, characterised in that it additionally contains an oxidation agent.

4. Process for the detection of substances with hydrolase activity in a sample by mixing of the sample with a hydrolase substrate and an oxidation agent and evaluation of the resulting colour intensity, characterised in that at least one compound according to claim 1 is used as hydrolase substrate.

5. Process according to claim 4, characterised in that the evaluation takes place visually.

6. Process for the preparation of compounds according to claim 1, characterised in that compounds of the formula II

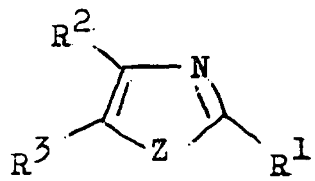

(II)

in which Z signifies oxygen, sulphur or the group N-$R^4$, $R^1$, $R^2$ and $R^3$ can be the same or different and signify hydrogen, an alkyl or aralkyl group or an aromatic radical, $R^4$ represents hydrogen or an alkyl group and in which one or more of the radicals $R^1$, $R^2$ and $R^3$ signify an aromatic radical substituted by the group -O-H, in which O represents oxygen, are reacted with compounds of the formula III

X - Y (III)

in which X has the meaning given in claim 1 and Y signifies a reactive group.

7. Use of compounds according to claim 1 for the detection of substances with hydrolase activity.

8. Test strips for the detection of an analyte with the use of a hydrolase-labelled compound and of a hydrolase substrate, characterised in that, as hydrolase substrate, there is used a compound of the formula I which is impregnated on an absorbent carrier.

**Revendications**

1. Composé de formule I

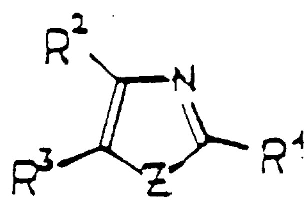

(I)

dans laquelle

| | |
|---|---|
| Z | représente l'oxygène, le soufre ou le groupe N-$R^4$ |
| $R^1$, $R^2$ et $R^3$ | peuvent être identiques ou différents et représentent un atome d'hydrogène, un groupe alkyle ou aralkyle ou un reste aromatique, |
| $R^4$ | représente un atome d'hydrogène ou un groupe alkyle |

et dans laquelle un ou plusieurs des restes $R^1$, $R^2$ et $R^3$ représentent un reste aromatique substitué par le groupe

-O-X

où

| | |
|---|---|
| O | représente l'oxygène et |
| X | représente un reste glycosyle, phosphate ou acyle. |

2. Moyen pour la détection, dans un échantillon, de substances ayant une activité d'hydrolase, caractérisé en ce qu'il contient au moins un composé selon la revendication 1.

3. Moyen selon la revendication 2, caractérisé en ce qu'il contient en outre un agent oxydant.

4. Procédé de détection, dans un échantillon, de substances ayant une activité d'hydrolase, consistant à mélanger l'échantillon avec un substrat d'hydrolase et un agent oxydant et à évaluer l'intensité de la coloration formée, caractérisé en ce que, comme substrat d'hydrolase, on utilise au moins un composé selon la revendication 1.

5. Procédé selon la revendication 4, caractérisé en ce que l'évaluation est réalisée visuellement.

6. Procédé de préparation de composés selon la revendication 1, caractérisé en ce que l'on fait réagir des composés de formule II

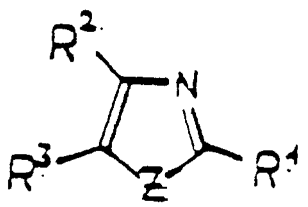

(II),

dans laquelle

Z représente l'oxygène, le soufre ou le groupe $N-R^4$

$R^1$, $R^2$ et $R^3$ peuvent être identiques ou différents et représentent un atome d'hydrogène, un groupe alkyle ou aralkyle ou un reste aromatique,

$R^4$ représente un atome d'hydrogène ou un groupe alkyle

et dans laquelle un ou plusieurs des restes $R^1$, $R^2$ et $R^3$ représentent un reste aromatique substitué par le groupe

-O-H

où

O représente l'oxygène

avec des composés de formule III

X-Y (III),

où X a la signification mentionnée ci-dessus et Y représente un groupe réactif.

7. Utilisation de composés selon la revendication 1, pour la détection de substances ayant une activité d'hydrolase.

8. Bandes de test pour la détection d'un analyte, utilisant un composé marqué par une hydrolase et un substrat d'hydrolase, caractérisé en ce que, comme substrat d'hydrolase, on utilise un composé de formule I qui imprègne un support absorbant.

Fig 1

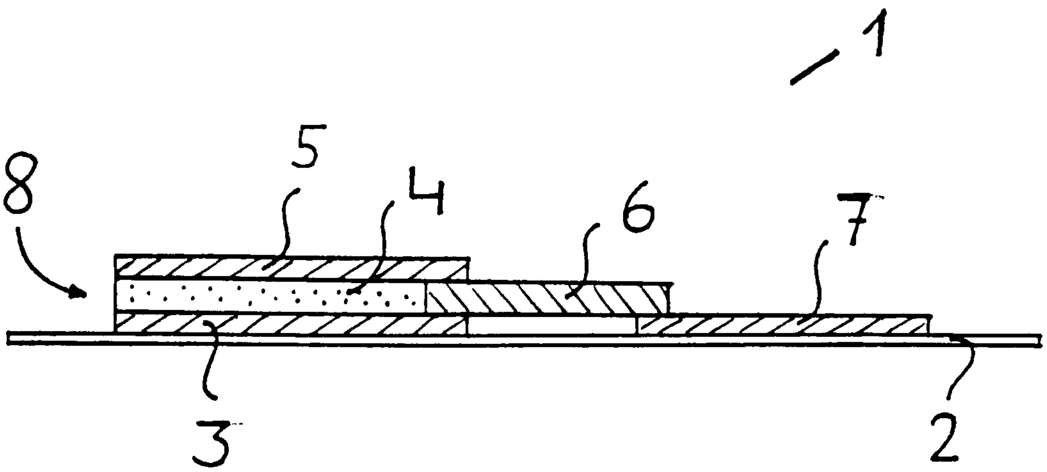

Fig 2